# EUROPÄISCHE PATENTSCHRIFT

(11) **EP 2 605 710 B1**
(45) Veröffentlichungstag und Bekanntmachung des Hinweises auf die Patenterteilung: **21.10.2015**
(21) Anmeldenummer: 11804924.6
(22) Anmeldetag: 18.08.2011
(51) Int. Cl.: A61B 17/00, G01L 5/22

(54) **MINIMALINVASIVES INSTRUMENT**
MINIMALLY INVASIVE INSTRUMENT
INSTRUMENT MINIMALEMENT INVASIF

(30) Priorität: 18.08.2010 DE 102010034719; 18.08.2010 DE 102010034717; 18.08.2010 DE 102010034712
(43) Veröffentlichungstag der Anmeldung: 26.06.2013
(73) Patentinhaber: Deutsches Zentrum für Luft- und Raumfahrt e.V., 51147 Köln (DE)
(72) Erfinder: STROHMAYR, Michael, 81667 München (DE); LANTERMANN, Sophie, 81679 München (DE); FRÖHLICH, Florian, Alexander, 80797 München (DE)
(74) Vertreter: Rasch, Michael
(86) Internationale Anmeldenummer: PCT/DE2011/001614
(87) Internationale Veröffentlichungsnummer: WO 2012/037917

(56) Entgegenhaltungen:
- DE-A1- 4 332 580
- US-A- 4 492 949
- US-A1- 2007 227 267

## Beschreibung

Die Erfindung betrifft ein minimalinvasives Instrument, mittels dem durch einen Zugangskanal hindurch ein Vorgang durchführbar ist, mit einem in den Zugangskanal einführbaren distalen Ende und einem dem distalen Ende zugeordneten taktilen Sensor, mittels dem während des Vorgangs taktile Stimuli erfassbar sind.

Minimalinvasive Instrumente sind bekannt. Diese weisen üblicherweise ein distales Ende auf, das durch einen vergleichsweise kleinen Zugangskanal hindurchgeführt werden kann, wobei mittels des distalen Endes ein Vorgang durchgeführt werden kann. Der Zugangskanal kann beispielsweise von einem Trokar gebildet sein. Solche minimalinvasiven Instrumente können beispielsweise für Untersuchungen und/oder minimalinvasive Eingriffe verwendet werden. Solche minimalinvasiven Instrumente können handgeführt oder robotergestützt geführt sein. Die DE 10 2007 037 262 B3 betrifft einen Kraftmomentsensor zum Messen von mindestens drei orthogonalen Belastungen. Zum Messen von mindestens drei orthogonalen Belastungen sind stabartige Elemente in Form von Glasfasern ohne Trägerstruktur vorgesehen, die in Plattenform fixiert sind. Alternativ und/oder zusätzlich bilden stabartige Abschnitte eine fortlaufende Glasfaser oder einige wenige Glasfasern, welche wellenförmig um einen gedachten Zylinder gewickelt sind. Auf den Glasfasern kann eine Ummantelung aufgebracht sein. Der Kraftmomentsensor kann bei Greifeinrichtungen Verwendung finden, die in einer Medizintechnik eingesetzt werden. Außerdem kann der Kraftmomentensensor bei Instrumenten Verwendung finden, die in einer minimalinvasiven Chirurgie eingesetzt werden.

Aus der DE 43 32 580A1 ist eine Vorrichtung zur Nachbildung oder Simulation des Tastsinns für medizinische, insbesondere chirurgische Zwecke wie Eingriffe mittels medizinischer Instrumente bekannt. Diese Vorrichtung zeichnet sich dadurch aus, dass wenigstens ein kraft- oder druck- oder wegeempfindliches Sensorarray und Aktorarray am Instrument zu einer "abfühlenden" oder "taktilen" Einheit derart zusammengefasst sind, daß das Sensorarray das Aktorarray steuert.

Aus der US 2007/0227267 A1 ist ein taktiler Sensor mit einem festen Körper der an seine gekrümmten Oberfläche mehrere Elektroden aufweist, zumindest einer deformierbaren Schicht, die mit der Oberfläche verbunden ist, und einer zwischen dem Körper und der deformierbaren Schicht angeordnetes Volumen mit einem deformierbaren Material.

Auch aus der US 4,492,949 A ist ein taktiler Sensor bekannt, der in diesem Fall einen Schichtverbund aus drei Schichten aufweist, umfassend eine flexible Top-Schicht, eine Grund-Schicht und eine Mittelschicht, wobei die Mittelschicht eine Vielzahl von verteilten elektrisch leitenden Stellen aufweist.

Aufgabe der Erfindung ist es, ein verbessertes minimalinvasives Instrumentieren zu ermöglichen, insbesondere dabei ein verbessertes taktiles Sensieren zu ermöglichen.

Die Aufgabe ist bei einem minimalinvasiven Instrument aufweisend einen vergrößerbaren Sensorkopf mit einem elastische Eigenschaften aufweisenden flächigen Sensorelement dadurch gelöst, dass das Sensorelement ein Folienelement mit einer ersten Schicht und einer mittels Abstandshaltern von der ersten Schicht beabstandeten zweiten Schicht aufweist, wobei das Sensorelement zwischen den Schichten des Folienelements angeordnete, dehnungssensitive, polymerbasierte Widerstandselemente zum Aufnehmen taktiler Stimuli aufweist, und der Sensorkopf eine elektromechanische Schnittstelle aufweist, mittels der der Sensorkopf lösbar fest mit dem Instrument verbindbar ist.

Der Sensorkopf weist einen taktilen Sensor auf. Der taktile Sensor weist das Sensorelement auf. Vorteilhaft können die taktilen Stimuli mittels den polymerbasierten Widerstandselementen sensiert beziehungsweise aufgenommen werden, wobei die taktilen Stimuli zu Dehnungen der polymerbasierten Widerstandselemente führen beziehungsweise in solche wandelbar sind. Die Dehnungen der polymerbasierten Widerstandselemente wiederum bewirken eine Änderung einer Leitfähigkeit, die wiederum durch eine entsprechende Beschaltung einer Auswerteeinheit in ein elektrisches Widerstandssignal, beispielsweise eine Spannung und/oder einen Strom, wandelbar ist. Das Folienelement weist die elastisch verformbare erste Schicht mit einer ersten Oberfläche, und die elastisch verformbare zweite Schicht mit einer zweiten Oberfläche auf, wobei die erste und die zweite Oberfläche einander zugewandt, durch zwischen der ersten und zweiten Oberfläche ausgebildete, vereinzelt angeordnete, elastisch verformbare Abstandshalter voneinander beabstandet angeordnet und mit einander verbunden sind, und zwischen den Abstandshaltern und der ersten und zweiten Oberfläche definierte Zwischenräume/Hohlräume ausgebildet sind. Mittels des minimalinvasiven Instruments ist durch einen Zugangskanal hindurch ein Vorgang durchführbar. Es weist ein in den Zugangskanal einführbares distales Ende und einen dem distalen Ende zugeordneten taktilen Sensor des Sensorkopfs auf, mittels dem während des Vorgangs taktile Stimuli erfassbar sind. Es ist vorgesehen, dass der taktile Sensor das elastische Eigenschaften aufweisende flächige Sensorelement mit einer durch den Zugangskanal durchführbaren und danach bereitstellbaren taktil sensitiven Oberfläche aufweist, wobei in einem bereitgestellten Zustand eine flächige Ausdehnung der taktil sensitiven Oberfläche ein Maß eines Zugangskanalquerschnitts des Zugangskanals übersteigt. Vorteilhaft kann die taktil sensitive Oberfläche nach dem Einführen durch den vergleichsweise engen Zugangskanal derartig bereitgestellt werden, dass die flächige Ausdehnung das Maß des Zugangskanalquerschnitts des Zugangskanals übersteigt. Unter einem Maß des Zugangskanalquerschnitts kann beispielsweise eine Höhe, eine Breite, eine Fläche, im Fall eines kreisförmigen Zugangskanals ein Durchmesser und/oder ein ähnliches Maß verstanden werden. Ferner kann unter Übersteigen verstanden werden, dass die taktil sensitive Oberfläche in dem bereitgestellten Zustand den Zugangskanal nicht kollisionsfrei passieren kann. Vorteilhaft kann trotz des vergleichsweise kleinen Zugangskanals die vergleichsweise große taktil sensitive Oberfläche bereitgestellt werden, wobei vorteilhaft die taktilen Stimuli gleichzeitig von einer größeren Oberfläche aufgenommen werden können. Unter dem Vorgang kann beispielsweise eine Palpation von Gewebe verstanden werden, wobei dazu der taktile Sensor beziehungsweise die taktil sensitive Oberfläche des taktilen Sensors mit dem zu palpierenden Gewebe in einen taktile Stimuli übertragenden Anlagekontakt bringbar ist. Dies kann beispielsweise durch eine entsprechende Manipulation des minimalinvasiven Instruments, beispielsweise handgeführt und/oder roboterunterstützt geführt, erfolgen. Alternativ und/oder zusätzlich kann unter dem Vorgang ein Anheben und/oder ein Positionieren von Gewebe, insbesondere eines Organs, verstanden werden. Vorteilhaft können während des Vorgangs taktile Stimuli zum Palpieren des Gewebes und/oder taktile Stimuli zum Überwachen eines Anpressdrucks während des Anhebens und/oder Positionierens des Gewebes, insbesondere Organs, erfolgen. Unter dem distalen Ende zugeordnet, kann verstanden werden, dass der taktile Sensor an dem distalen Ende angeordnet ist. Insbesondere kann der taktile Sensor das distale Ende ausmachen und/oder das distale Ende die taktil sensitive Oberfläche des taktilen Sensors aufweisen.

Bei einem Ausführungsbeispiel des Instruments ist vorgesehen, dass die taktil sensitive Oberfläche mittels einer Spannvorrichtung bereitstellbar ist. Vorteilhaft kann das Sensorelement des taktilen Sensors mittels der Spannvorrichtung nach dem Einführen beziehungsweise Durchführen durch den Zugangskanal so aufgespannt werden, dass die vergleichsweise große taktil sensitive Oberfläche bereitgestellt ist.

Bei einem weiteren Ausführungsbeispiel des Instruments ist vorgesehen, dass das Sensorelement einen Hohlraum zumindest teilweise fluiddicht umschließt. Vorteilhaft kann der Hohlraum während des Durchführens durch den Zugangskanal und aufgrund der elastischen Eigenschaften des Sensorelements zurückweichen, wobei durch Rückstellkräfte nach dem Durchschieben durch den Zugangskanal die vergleichsweise große taktil sensitive Oberfläche bereitgestellt ist.

Bei einem weiteren Ausführungsbeispiel des Instruments ist vorgesehen, dass die Behandlungsvorrichtung eine Fluidstromquelle aufweist, die dem Hohlraum mittels eines Fluidpfads zugeordnet ist. Vorteilhaft kann es sich bei der Fluidstromquelle um eine ohnehin bei minimalinvasiven Instrumenten vorgesehene Pumpe, insbesondere eine bidirektionale Pumpe, Saugpumpe und/oder Druckpumpe, handeln. Vorteilhaft kann mittels der Fluidstromquelle der Hohlraum wahlweise aufgepumpt oder entleert werden. Die Fluidstromquelle kann zum Fördern eines beliebigen Fluids, beispielsweise einer Flüssigkeit, insbesondere Wasser, ausgelegt sein. Alternativ und/oder zusätzlich kann es sich bei dem Fluid um Luft handeln. Unter der Fluidstromquelle kann alternativ ein unter Überdruck oder Unterdruck stehender Fluidspeicher oder eine Fluidquelle, z.B. ein Druckgasanschluss, ein Wasseranschluss oder Ähnliches verstanden werden. Der Begriff Fluidstromquelle ist daher breit auszulegen, insbesondere als Quelle eines Fluidstroms, wobei eine Richtung des Fluidstroms beziehungsweise ein die Richtung angebendes Druckgefälle, das den Fluidstrom antreibt, nicht festgelegt ist.

Bei einem weiteren Ausführungsbeispiel des Instruments ist vorgesehen, dass der Hohlraum teilweise von einem Schaft und teilweise von dem Sensorelement fluiddicht umschlossen ist. Vorteilhaft kann das Sensorelement eine flächige Ausdehnung, insbesondere ähnlich einer Folie, aufweisen, die dem distalen Ende fluiddicht zugeordnet ist. Dadurch entsteht innerhalb einer Außenfläche des Schafts und des Sensorelements der Hohlraum. Vorteilhaft kann dieser Hohlraum mit dem Fluid unter einem bestimmten Druck gefüllt werden, wobei vorteilhaft sich das Sensorelement entsprechend dabei auftretenden hydrostatischen Kräften entfaltet, wobei dadurch die vergleichsweise große taktil sensitive Oberfläche bereitgestellt ist.

Bei einem weiteren Ausführungsbeispiel des Instruments ist vorgesehen, dass der Fluidpfad in den Hohlraum mündet. Vorteilhaft kann über den Fluidpfad das Fluid in den Hohlraum eingebracht oder wahlweise aus diesem herausgebracht, insbesondere von diesem abgesaugt werden.

Die Zwischenräume sind bevorzugt mit einem fluiden Medium gefüllt, wobei unter einem fluiden Medium vorliegend eine Substanz verstanden wird, die einer beliebig langsamen Scherung keinen Widerstand entgegensetzt und somit eine endliche Viskosität aufweist. Fluide Medien umfassen daher insbesondere Gase und Flüssigkeiten, aber auch Gele. Weiterhin können die Zwischenräume des Folienelements insgesamt zu einer Umgebung des Folienelements hin offen oder geschlossen sein, d.h. im ersten Fall kann das fluide Medium aus dem Folienelement entweichen oder in dieses eindringen, insbesondere ist der Außendruck (bspw. der Luftdruck) gleich dem Innendruck in den Zwischenräumen, im zweiten Fall ist das fluide Medium in den Zwischenräumen eingeschlossen. Die Wahl des fluiden Mediums und die Ausführungsform (nach Außen abgeschlossene/offene Zwischenräume) beeinflussen die elastischen Eigenschaften des Folienelements und können den Anforderungen entsprechend ausgewählt werden.

Durch die Verwendung vereinzelt angeordneter, elastisch verformbarer Abstandshalter zwischen den zwei Schichten des Folienelements wird das Materialvolumen, das bei einer Einwirkung eines mechanischen Krafteintrages verformt werden muss, reduziert. Weiterhin ergibt sich durch die bekannten Zwischenräume eine geometrisch bekannte Dichteverteilung im Folienelement, so dass das Hystereseverhalten des Folienelements bei mechanischer Verformung beherrschbar ist. Zusätzlich kann ein anisotropes elastisches Verformungsverhalten eingestellt werden, d.h. das zu verformende Volumen kann frei definiert und im Folienelement, durch entsprechende Anordnung der Abstandshalter, angeordnet werden. Bei Einsatz des Folienelements bei dem taktilen Sensor kann so die Empfindlichkeit des Sensors an die zu lösende Aufgabe angepasst werden, bspw. hohe Empfindlichkeit gegenüber senkrechten Kräften bei gleichzeitig geringer Empfindlichkeit gegenüber horizontalen Kräften und umgekehrt. Vorteilhaft können die Abstandshalter einen von einer Kreisform abweichenden Querschnitt aufweisen, beispielsweise ovalförmig, polygonförmig oder rechteckig. Auf einzelne Abstandshalter einwirkende Scherkräfte - verursacht durch einen taktilen Stimulus - erfahren dadurch unterschiedlich starke und richtungsabhängige Gegenkräfte. Dadurch kann vorteilhaft neben anderen Maßnahmen auch das anisotrope Verhalten erzielt werden.

Das Folienelement ist neben taktilen Sensoren auch für passive/ aktive Schwingungsdämpfer, bei Greifern oder Greifwerkzeugen verwendbar. Werden die Zwischenräume mit einer elektro-rheologischen Flüssigkeit gefüllt, kann ein flächiges Dämpfungselement mit lokal einstellbarem Dämpfungsverhalten erstellt werden. Werden die Abstandshalter z.B. aus elektro-aktiven Polymeren hergestellt, kann ein aktives flächiges Dämpfungs- / Aktorelement erstellt werden.

Das Folienelement ist durch folgende Schritte herstellbar: Bereitstellen einer elastisch verformbaren ersten Schicht mit einer ersten Oberfläche, Aufbringen erster Abstandshalter aus einem aushärtbaren Polymermaterial in einer ersten Anordnung auf der ersten Oberfläche, Bereitstellen einer elastisch verformbaren zweiten Schicht mit einer zweiten Oberfläche, Fügen der zweiten Oberfläche mit den ersten Abstandshaltern an deren von der ersten Oberfläche abgewandten Enden, Auseinanderbewegen der ersten und zweiten Schicht so, dass die erste und zweite Oberfläche einen vorgegebenen Abstand voneinander aufweist und die ersten Abstandshalter die erste und zweite Oberfläche verbinden, Aushärten der ersten Abstandshalter, wobei das Polymermaterial der ersten Abstandshalter nach dem Aushärten elastisch verformbar ist und die ersten Abstandshalter in einem mechanisch unbelasteten Zustand die erste und die zweite Oberfläche in einem Abstand A beabstanden. Die Abstandshalter sind bevorzugt vereinzelt angeordnet. Sie können beliebig geformt (linienförmig, sternförmig, punktförmig, etc.), insbesondere auch zusammenhängend und/oder als in sich geschlossene Formen, bspw. als Kreis oder Oval etc. ausgebildet sein.

Die bereitgestellte erste und zweite Schicht besteht jeweils bevorzugt aus einem Polymermaterial, beispielsweise einem Thermoplastmaterial, insbesondere aus einem Silikonmaterial und weist bevorzugt eine Schichtdicke von < 15 mm, < 10mm, < 5 mm, < 2 mm, < 1 mm, < 0,5 mm, < 0,1 mm, oder < 0,05 mm auf. Die Schichtdicke der ersten und zweiten Schicht kann natürlich nach Bedarf unterschiedlich gewählt werden.

Das aushärtbare Polymermaterial der ersten Abstandshalter ist bevorzugt ebenfalls ein Thermoplastmaterial , oder ein Silikonmaterial, wobei unter dem Begriff "aushärtbar" vorliegend verstanden wird, dass das Polymermaterial der ersten Abstandshalter im Zeitpunkt des Aufbringens auf die erste Oberfläche noch nicht vollständig vernetzt, bzw. nicht vollständig vulkanisiert bzw. nicht vollständig ausgehärtet bzw. abgekühlt ist. Das Aufbringen der ersten Abstandshalter bzw. des aushärtbaren Polymermatrerials erfolgt bevorzugt mittels eines Druck-, Press-, Gieß-, Spritzguss-, Rakel- oder Kalandrierverfahrens. Die erste Oberfläche und die aufgebrachten ersten Abstandshalter verbinden sich nach deren Aufbringen, ggf. ist hierfür eine chemische oder thermische Vorbehandlung (Erwärmen) der ersten Oberfläche erforderlich.

Zur Einstellung eines vorbestimmten Verformungsverhaltens des Folienelements können je nach Anforderung für die erste, die zweite Schicht und die ersten Abstandshalter gleiche oder unterschiedliche elastische Materialien verwendet werden. Das richtungsabhängige (anisotrope) Verformungsverhalten des Folienelements lässt sich weiterhin durch die Wahl der ersten Anordnung der ersten Abstandshalter auf die erste Oberfläche einstellen. Vorzugsweise weist die Anordnung der ersten Abstandshalter eine trapezförmige, ringförmige, elliptische oder rechteckige, insbesondere quadratische Geometrie auf. Natürlich sind je nach Anforderungen beliebige weitere erste Anordnungen realisierbar.

Nachdem die ersten Abstandshalter auf die erste Oberfläche aufgebracht sind und sich mit dieser verbunden haben, wird vorteilhaft die zweite elastisch verformbare Schicht mit einer zweiten Oberfläche bereitgestellt. Anschließend erfolgt das Fügen der zweiten Oberfläche mit den ersten Abstandshaltern an deren von der ersten Oberfläche abgewandten Enden. Beim Fügen geht das Polymermaterial der ersten Abstandshalter eine feste Verbindung mit der zweiten Oberfläche ein. Hierzu ist ggf. ebenfalls eine physikalische, chemische oder thermische Vorbehandlung der zweiten Oberfläche erforderlich. Nach diesem Verfahrensschritt ist eine Art "Sandwichfolie" bzw. ein Folienverbund erzeugt, der die erste, die zweite Schicht und dazwischen angeordneten erste Abstandshalter aufweist. Zwischen den Abstandshaltern, der ersten und der zweiten Oberfläche sind Zwischenräume ausgebildet.

Nachdem sich die ersten Abstandshalter mit der zweiten Oberfläche verbunden haben, werden die erste und die zweite Schicht auseinander bewegt, sodass die erste und die zweite Oberfläche einen vorgegebenen Abstand voneinander aufweisen und die ersten Abstandshalter die erste und zweite Oberfläche verbinden. Der vorgegebene Abstand ist in jedem Fall so zu wählen, dass die ersten Abstandshalter die erste und zweite Oberfläche weiterhin verbinden, d.h. nicht reißen oder sich von einer oder beiden der ersten und zweiten Oberflächen lösen. Durch das Auseinenderbewegen der ersten und zweiten Schicht werden die ersten Abstandshalter in ihren Außenformen verändert. Typischerweise stellt sich hierbei eine zylinderförmige oder einschalig hyperboloide Außenkontur der ersten Abstandshalter ein. Weiterhin wird durch das Auseinanderbewegen die Größe der zwischen den ersten Abstandhaltern und den ersten und zweiten Oberfläche ausgebildeten Zwischenräume eingestellt. Über das Einstellen der Form der ersten Abstandshalter und der Größe der Zwischenräume kann wiederum das richtungsabhängige (anisotrope) Verformungsverhalten des Folienelements vorgegeben bzw. eingestellt werden.

Das Auseinanderbewegen der ersten und der zweiten Schicht kann beispielsweise mittels zweier perforierter Platten erfolgen, die zur Erzeugung von Unterdruck auf einer ihrer jeweiligen Oberflächen an eine Unterdruckpumpe angeschlossen sind. Diese Oberflächen werden in Kontakt mit zur ersten bzw. zweiten Oberfläche jeweils gegenüberliegenden Oberflächen der ersten bzw. zweiten Schicht gebracht. Die erste und zweite Schicht haften aufgrund des Unterdrucks an den perforierten Platten und können mittels eines entsprechenden Auseinanderbewegens der Platten gezielt voneinander beabstandet werden.

Nach dem Auseinanderbewegen erfolgt das Aushärten der ersten Abstandshalter, wobei das Polymermaterial der Abstandshalter nach dem Aushärten elastisch verformbar ist und die Abstandshalter in einem mechanisch unbelasteten Zustand die erste und die zweite Oberfläche in einem Abstand A beabstanden. Der Begriff "Aushärten" wird vorliegend im Sinne von "vernetzen", "vulkanisieren", "aushärten", oder "abkühlen" je nach verwendetem Polymermaterial verwendet. Nach dem Aushärten ist das Polymermaterial der ersten Abstandshalter elastisch verformbar und im Wesentlichen formstabil.

In einer vorteilhaften Weiterbildung des Verfahrens wird der Verbund aus Abstandshaltern und erster und zweiter Schicht vor dem Auseinanderbewegen erwärmt und nach dem Auseinanderbewegen gekühlt.

Das Folienelement, d.h. der Schichtverbund aus erster und zweiter Schicht mit den dazwischen angeordneten ersten Abstandshaltern, weist bevorzugt eine (Folienelement-) Dicke von < 50 mm, < 25 mm, <10 mm, < 5 mm, < 2 mm, < 1 mm, < 0,5 mm oder < 0,1 mm auf. Weiterhin beträgt der Abstand A bevorzugt < 15 mm, < 10mm, < 5 mm, < 3 mm, < 2 mm, < 1 mm, < 0,5 mm, < 0,2 mm. Natürlich hängt die Wahl der Schichtdicken der ersten und zweiten Schicht, des Abstandes A und der Form und Masse der einzelnen ersten Abstandshalter voneinander und von der vorliegenden Aufgabenstellung ab, so dass deren Wahl nicht beliebig aber einem Fachmann einfach möglich ist.

Das mit dem Verfahren erzeugte elastisch verformbare Folienelement weist zwei Schichten (die erste und die zweite Schicht) auf, die durch dazwischen vereinzelt angeordnete, entsprechend geformte Abstandshalter voneinander im Abstand A beabstandet sind. Die Eigenschaften der mechanischen elastischen Verformbarkeit des Folienelements können durch geeignete Wahl der beteiligten elastischen Materialien, der ersten Anordnung der ersten Abstandshalter, des Abstandes A sowie der durch das Auseinanderbewegen erzeugten Form der ersten Abstandshalter eingestellt werden.

Gemäß einer Alternative ist das Folienelement durch folgende Schritte herstellbar: Bereitstellen einer elastisch verformbaren ersten Schicht mit einer ersten Oberfläche, Aufbringen vereinzelter zweiter Abstandshalter aus einem aushärtbaren Polymermaterial in einer zweiten Anordnung auf der ersten Oberfläche, Bereitstellen einer elastisch verformbaren zweiten Schicht mit einer zweiten Oberfläche, Aufbringen vereinzelter dritter Abstandshalter aus einem aushärtbaren Polymermaterial in einer zur zweiten Anordnung spiegelsymmetrischen dritten Anordnung auf der zweiten Oberfläche, Ausrichten der ersten und zweiten Schicht, so dass sich die auf der ersten und auf der zweiten Oberfläche aufgebrachten Anordnungen der zweiten und dritten Abstandshalter deckungsgleich gegenüberliegen, Fügen der sich jeweils deckungsgleich gegenüberliegenden zweiten und dritten Abstandshalter an ihren jeweiligen freien Enden, Auseinanderbewegen der ersten und zweiten Schicht so, dass die erste und die zweite Oberfläche einen vorgegebenen Abstand voneinander aufweist und die Abstandshalter die erste und zweite Oberfläche verbinden, und Aushärten der Abstandshalter, wobei das Polymermaterial der Abstandshalter nach dem Aushärten elastisch verformbar ist und die Abstandshalter in einem mechanisch unbelasteten Zustand die erste und die zweite Oberfläche in einem Abstand A beabstanden.

Im Unterschied zum davor beschriebenen Verfahren werden bei dieser Verfahrensalternative die Abstandshalter nicht nur auf die Oberfläche der ersten Schicht aufgebracht, sondern die endgültigen Abstandhalter ergeben sich aus zwei Teilen: den zweiten und dritten Abstandshaltern, die in zueinander spiegelbildlicher Anordnung auf die erste und zweite Oberfläche aufgebracht werden. Vorteilhaft ist bei diesem Verfahren somit das Ausrichten der ersten und zweiten Schicht erforderlich, so dass sich die auf der ersten und auf der zweiten Oberfläche aufgebrachten Anordnungen der zweiten und dritten Abstandshalter deckungsgleich gegenüberliegen. Das Verbinden der bereitgestellten ersten und zweiten Schicht ergibt sich in diesem Fall durch das Fügen der sich jeweils deckungsgleich gegenüberliegenden zweiten und dritten Abstandshalter an ihren jeweiligen freien Enden. Bevorzugt werden die Abstandshalter vor dem Fügen thermisch, chemisch, mechanisch, mit elektromagnetischer Strahlung, mit Plasma oder mit Teilchenstrahlung Fügen behandelt, um das Fügen der Abstandshalter und/oder eine Formänderung der Abstandshalter während des Auseinanderbewegens der ersten und zweiten Schicht zu befördern. In einer Verfahrensvariante erfolgt das Fügen der zweiten und der dritten Abstandshalter unter mechanischem Vibrieren der Abstandshalter bzw. der mit diesen jeweils verbundnen ersten oder zweiten Schicht. Die zweiten und die dritten Abstandshalter weisen bevorzugt eine identische Form auf, insbesondere eine halbkugelförmige, kegelförmige, tropfenförmige, zylinderförmige, quaderförmige oder eine einschalige hyperboloide Außenkontur (-form) auf. Auch in dieser Verfahrensalternative wird bevorzugt vor dem Auseinanderbewegen der ersten und zweiten Schicht der Verbund aus Abstandshaltern und erster und zweiter Schicht erwärmt und nach dem Auseinanderbewegen gekühlt wird oder abkühlt.

Unter Beachtung des genannten Unterschieds lassen sich die Ausführungen und Erläuterungen zum Verfahren gemäß der ersten Alternative des Verfahrens auf das Verfahren gemäß der zweiten Alternative analog übertragen. Dabei entsprechen die aus zweiten und dritten Abstandshaltern zusammengesetzten Abstandshalter den ersten Abstandshaltern. Auf die entsprechenden Beschreibungsteile wird hiermit verwiesen. Natürlich lassen sich den vorgestellten Verfahren gemäß der ersten und zweiten alternative Folienelemente mit mehreren, mittels Abstandshaltern beabstandeten Schichten herstellen. Hierzu wird das jeweilige Verfahren mehrfach hintereinander angewendet, wobei bspw. die bereitgestellte erste Schicht bereits ein aus zwei oder mehr Schichten bestehendes Folienelement ist.

Bei dem Instruments ist vorgesehen, dass der taktile Sensor dem distalen Ende oder der taktile Sensor zusammen mit dem distalen Ende an einer elektromechanischen Schnittstelle des Instruments mit einem übrigen Teil des Instrument lösbar verbindbar ist. So können der taktile Sensor beziehungsweise der Sensorkopf bedarfsweise mit dem übrigen Teil des Instrument zusammengefügt werden. Dadurch kann das Instrument auch anderweitig, also ohne den taktilen Sensor verwendet werden. Unter der elektromechanischen Schnittstelle kann eine elektrische und fluidische Steckverbindung verstanden werden.

Bei einem weiteren Ausführungsbeispiel des Instruments ist vorgesehen, dass der taktile Sensor Bereiche mit unterschiedlich taktilen Auflösungen aufweist. Unter einem Bereich kann eine Teilfläche der taktil sensitiven Oberfläche des Sensorelements verstanden werden. Vorteilhaft können Bereiche mit hoher und mit niedriger taktiler Auflösung vorgesehen sein, wobei in Bereichen, in denen eine besonders hohe Auflösung erforderlich ist, diese erzielbar ist, ohne dass dadurch in Bereichen, in denen eine niedrigere taktile Auflösung erforderlich ist, unnötig hoher Messaufwand getrieben wird. Das Sensorelement mit unterschiedlichen taktilen Auflösungen weist eine nichtleitende, elastisch verformbare erste Schicht mit einer ersten Oberfläche, eine nichtleitende elastisch verformbare zweite Schicht mit einer zweiten Oberfläche, wobei die erste und die zweite Oberfläche einander zugewandt und durch zwischen der ersten und zweiten Oberfläche ausgebildete, elastisch verformbare Abstandshalter voneinander beabstandet angeordnet und miteinander verbunden sind, eine oder mehrere elastisch verformbare, elektrisch leitende erste Leitungen, die auf oder an der ersten Oberfläche angeordnet/fixiert sind, und elastisch verformbare, elektrisch leitende zweite Leitungen, die auf oder an der zweiten Oberfläche angeordnet/fixiert sind, wobei sich erste und die zweite Leitungen an Kreuzungsstellen kreuzen, auf. Bevorzugt sind die ersten/zweiten Leitungen in Bereichen der ersten/zweiten Oberfläche zwischen den Abstandshaltern angeordnet. Dies gilt insbesondere für die Kreuzungsstellen. Die ersten/zweiten Leitungen können jedoch auch in Abschnitten bzw. teilweise unterhalb der Abstandshalter verlaufen. Die ersten/zweiten Leiter können vorteilhaft die Widerstandselemente bilden.

Das vorteilhafte Sensorelement basiert auf dem vorab beschriebenen Folienelement. Dieses ist zum Erzielen der taktilen Auflösungen um erste und zweite elastisch verformbare, elektrisch leitende Leitungen ergänzt, die auf oder an der ersten bzw. zweiten Oberfläche angeordnet sind, wobei sich erste und zweite Leitungen an Kreuzungsstellen kreuzen. Durch diese Anordnung von elastisch verformbaren elektrisch leitenden Leitungen wird das Folienelement vorteilhaft zu dem flächigen taktilen Sensorelement, das die besondere elastische Verformbarkeit und den Aufbau des Folienelements dazu nutzt, die richtungsabhängige Auflösung bei der Erfassung taktiler Reize zu verbessern.

Die richtungsabhängige (anisotrope) elastische Verformbarkeit des Folienelements ist, durch eine geeignete Wahl der beteiligten elastischen Materialien (für die erste und zweite Schicht, für die Abstandshalter, sowie für die elastischen Leitungen), durch eine geeignete Wahl der Form der Abstandshalter, von deren Anordnung und von deren Masse, und durch eine geeignete Wahl des Abstandes mit der die erste und die zweite Oberfläche voneinander beabstandet sind, bei der Herstellung einstellbar. Mit der richtungsabhängigen elastischen Verformbarkeit des Folienelements wird vorliegend auch die richtungsabhängige Sensibilität des Sensorelements im Hinblick auf die Erfassung taktiler Reize festgelegt. So kann das Sensorelement je nach Anforderung an die Auflösung taktiler Reize entsprechend ausgeführt werden.

In einer einfachen Ausführungsform des Sensorelements sind die Abstandshalter zwischen der ersten und der zweiten Oberfläche als Gitterpunkte eines zweidimensionalen orthogonalen, insbesondere eines kartesischen Gitters angeordnet. Bevorzug sind dabei die ersten Leitungen zueinander parallel und die zweiten Leitungen zueinander parallel angeordnet, während die ersten und zweiten Leitungen zueinander orthogonal verlaufen. Natürlich sind beliebige weitere Anordnungen der Abstandshalter, beispielsweise mit konzentrischer, rechteckiger Geometrie, bei entsprechender Anordnung der ersten und zweiten Leitungen je nach Aufgabenstellung möglich. Weiterhin können die elastischen Abstandshalter selbst nahezu beliebige Formen annehmen, beispielsweise punktförmige, sternförmige, längliche, linienförmige, auch in sich geschlossene Formen, wie Kreise, Ellipsen etc.

Die flächige taktile Auflösung des Sensorelements bestimmt sich durch die Flächendichte der Kreuzungsstellen, insbesondere der zwischen den Abstandshaltern angeordneten Kreuzungsstellen. Jede Kreuzungsstelle stellt quasi eine taktile Sensorzelle dar. Ist die Flächendichte der Kreuzungsstellen in einem Flächenbereich groß, so ist die flächige taktile Auflösung des Sensorelements für diesen Flächenbereich hoch. Ist die Flächendichte der Kreuzungsstellen in einem Flächenbereich klein, so ist die flächige taktile Auflösung des Sensorelements für diesen Flächenbereich gering.

In einer Ausführungsform des Sensorelements sind die ersten und die zweiten Leitungen an den Kreuzungsstellen in einem mechanisch unbelasteten Zustand des Sensorelements voneinander beabstandet.

Der Begriff "mechanisch unbelasteter Zustand" meint, dass auf das Sensorelement keine taktilen Reize (Kräfte) einwirken.

Durch eine auf das Sensorelement aufgebrachte Kraft (taktiler Reiz) nähern sich die ersten und zweiten Leitungen an den Kreuzungsstellen einander an, was bei weiterer Annäherung bis zu ihrer Berührung, und bei noch weiterer Annäherung zu einer elastischen Verformung der ersten und zweiten Leitungen führen kann. Diese Annäherung der ersten und zweiten Leitungen an den Kreuzungsstellen hat kapazitive bzw. die elektrische Leitfähigkeit der ersten und zweiten Leitungen betreffende messbare Wirkungen, mittels denen die eingebrachten taktilen Reize in bekannter Weise ausgewertet werden können. Bei Verschwinden der aufgebrachten Kraft stellt sich der "mechanisch unbelastete Zustand" mit einer richtungsabhängig bekannten, beherrschbaren und bei der Auswertung der vorstehend angegebenen messbaren Wirkungen zu berücksichtigenden Hysterese ein.

In einer alternativen Ausgestaltung des Sensorelements berühren sich die ersten und die zweiten Leitungen an den Kreuzungsstellen bereits im mechanisch unbelasteten Zustand des Sensorelements. Durch das Aufbringen einer äußeren mechanischen Kraft (taktiler Reiz) auf das Sensorelement werden die ersten und zweiten Leitungen an den jeweils betroffenen Kreuzungsstellen nun elastisch verformt, was jeweils betroffenen eine messbare Änderung der elektrischen Leitfähigkeit der betroffenen Leitungen bewirkt.

Bevorzugt bestehen die erste und die zweite Schicht und die Abstandshalter aus einem Polymermaterial, insbesondere aus einem Silikonmaterial. Weiterhin bestehen die ersten und die zweiten Leitungen bevorzugt aus einem elektrisch leitenden Polymermaterial, wie cis-Polyacetylen (PA), trans-Polyacetylen (PA) oder Poly-Para-Phenylen (PPP). Alternativ können die ersten und zweiten Leitungen jeweils aus einem nicht-leitenden und elastisch verformbaren Leitungskörper mit darin eingelagerten elektrisch leitenden Partikeln bestehen.

Die erste und/oder die zweite Schicht weist/weisen bevorzugt eine Schichtdicke von < 30 mm, < 15 mm, < 10mm, < 5 mm, < 2 mm, < 1 mm, < 0,5 mm, < 0,1 mm, oder < 0,05 mm auf. Je nach Anwendung weist das Sensorelement bestehend aus erster und zweiter Schicht und dazwischen angeordneten Abstandshaltern eine Dicke von < 50 mm, < 25 mm, < 10 mm, < 5 mm, < 2 mm, < 1 mm, < 0,5 mm oder < 0,1 mm auf. Weiterhin beträgt der Abstand der ersten zur zweiten Oberfläche bevorzugt < 15 mm, < 10 mm, < 5 mm, < 3 mm, < 2 mm, < 1 mm, < 0,5 mm, < 0,2 mm, < 0,1 mm oder <0,05 mm. Die erste und die zweite Oberfläche können mit einem flächig konstanten Abstand voneinander beabstandet sein, dieser Abstand kann aber auch, je nach Anwendung und Aufgabenstellung flächig variieren. In einer besonders bevorzugten Ausführungsform sind die Abstandshalter als Gitterpunkte eines zweidimensionalen kartesischen Gitters angeordnet, das eine Gitterkonstante im Bereich von: < 10 mm, < 5 mm, < 3mm, < 1 mm, < 0,5 mm, < 0,1 mm, < 0,05 mm oder < 0,01 mm aufweist.

Die Zwischenräume zwischen den Abstandshaltern und der ersten und der zweiten Oberfläche sind bevorzugt von einem fluiden Medium ausgefüllt, wobei unter einem fluiden Medium vorliegend eine Substanz verstanden wird, die einer beliebig langsamen Scherung keinen Widerstand entgegensetzt und somit eine endliche Viskosität aufweist. Fluide Medien umfassen daher insbesondere Gase und Flüssigkeiten, aber auch Gele. Weiterhin können die Zwischenräume des Folienelements insgesamt zu einer Umgebung des Folienelements hin offen oder geschlossen sein, d.h. im ersten Fall kann das fluide Medium aus dem Folienelement entweichen oder in dieses eindringen, insbesondere ist der Außendruck (bspw. der Luftdruck) gleich dem Innendruck in den Zwischenräumen, im zweiten Fall ist das fluide Medium in den Zwischenräumen eingeschlossen. Die Wahl des fluiden Mediums und die Ausführungsform (nach Außen abgeschlossene/offene Zwischenräume) beeinflussen die elastischen Eigenschaften des Folienelements und können den Anforderungen entsprechend ausgewählt werden.

Eine besonders bevorzugte Weiterbildung des Sensorelements zeichnet sich dadurch aus, dass das Sensorelement aus mehreren übereinander angeordneten Sensorelementen gebildet ist, d.h. dass mehrere vorteilhaft mit elektrischen Leitungen ausgestatte Folienelemente übereinander angeordnet sind.

Als Beschaltung ist dem Sensorelement ein Auswertemodul beziehungsweise eine Auswerteeinheit zugeordnet, das/die mit den ersten und zweiten Leitungen verbindbar ist, wobei das Auswertemodul beziehungsweise die Auswerteeinheit ein Kapazitätsmessmodul, mit dem elektrische Kapazitätsänderungen an einzelnen Kreuzungspunkten der ersten und zweiten Leitungen ermittelbar sind, und/oder ein Widerstandsmessmodul, mit dem ein elektrischer Übergangswiderstand an einzelnen Kreuzungspunkten der ersten und zweiten Leitungen ermittelbar ist, und/oder ein Widerstandsmessmodul, mit dem elektrische Leitungswiderstände der einzelnen ersten und zweiten Leitungen ermittelbar sind, auf. Aus den ermittelten Kapazitätsänderungen bzw. Übergangswiderstandsänderungen bzw. Leitungswiderstandsänderungen lassen sich der Ort bzw. die betroffene Fläche des taktilen Reizes auf dem Sensorelement als auch der mit dem taktilen Reiz verbundene Krafteintrag ermitteln.

Ein vorteilhaftes Verfahren zum Herstellen des Sensorelements zur Erfassung taktiler Reize weist folgende Verfahrensschritte auf: Bereitstellen einer elastisch verformbaren ersten Schicht mit einer ersten Oberfläche, Aufbringen erster Abstandshalter aus einem aushärtbaren Polymermaterial in einer ersten Anordnung auf der ersten Oberfläche, Aufbringen einer oder mehrerer elastisch verformbarer, elektrisch leitender erster Leitungen auf oder an der ersten Oberfläche, Bereitstellen einer elastisch verformbaren zweiten Schicht mit einer zweiten Oberfläche, Aufbringen mehrerer elastisch verformbarer, elektrisch leitender zweiter Leitungen auf oder an der zweiten Oberfläche, Fügen der zweiten Oberfläche auf die ersten Abstandshalter, an deren von der ersten Oberfläche abgewandten Enden, wobei sich die ersten und zweiten Leitungen an Kreuzungsstellen kreuzen, Auseinanderbewegen der ersten und zweiten Schicht so, dass die erste und zweite Oberfläche einen vorgegebenen Abstand voneinander aufweist und die Abstandshalter die erste und zweite Oberfläche verbinden, Aushärten der Abstandshalter, wobei das Polymermaterial der Abstandshalter nach dem Aushärten elastisch verformbar bleibt und die Abstandshalter in einem mechanisch unbelasteten Zustand die erste und die zweite Oberfläche in einem Abstand A beabstanden.

In einer Weiterbildung des vorstehenden Verfahrens erfolgt zunächst das Aufbringen einer oder mehrerer elastisch verformbarer, elektrisch leitender erster Leitungen auf oder an der ersten Oberfläche und anschließend das Aufbringen der ersten Abstandshalter aus einem aushärtbaren Polymermaterial in einer ersten Anordnung auf der ersten Oberfläche, wobei die Abstandshalter auch teilweise auf die bereits vorhandenen ersten Leitungen aufgebracht werden können. Die Abstandshalter werden bevorzugt vereinzelt, d.h. sich nicht berührend, aufgebracht. Weiterhin werden die ersten und zweiten Leitungen bevorzugt derart auf die jeweiligen Oberflächen aufgebracht, dass sie im fertig hergestellten Sensorelement zwischen den Abstandshaltern angeordnet sind.

Ein vorteilhaftes alternatives Verfahren zum Herstellen des Sensorelements zur Erfassung taktiler Reize weist folgende Verfahrensschritte auf: Bereitstellen einer elastisch verformbaren ersten Schicht mit einer ersten Oberfläche, Aufbringen zweiter Abstandshalter aus einem aushärtbaren Polymermaterial in einer zweiten Anordnung auf der ersten Oberfläche, Aufbringen einer oder mehrerer elastisch verformbarer, elektrisch leitender erster Leitungen auf oder an der ersten Oberfläche, Bereitstellen einer elastisch verformbaren zweiten Schicht mit einer zweiten Oberfläche, Aufbringen dritter Abstandshalter aus einem aushärtbaren Polymermaterial in einer zur zweiten Anordnung spiegelsymmetrischen dritten Anordnung auf der zweiten Oberfläche, Aufbringen einer oder mehrerer elastisch verformbarer, elektrisch leitender zweiter Leitungen auf oder an der zweiten Oberfläche, Ausrichten der ersten und zweiten Schicht, so dass sich die auf der ersten und auf der zweiten Oberfläche aufgebrachten Anordnungen der zweiten und dritten Abstandshalter deckungsgleich gegenüberliegen, Fügen der sich jeweils deckungsgleich gegenüberliegenden zweiten und dritten Abstandshalter an ihren jeweiligen freien Enden, Auseinanderbewegen der ersten und zweiten Schicht so, dass die erste und die zweite Oberfläche einen vorgegebenen Abstand voneinander aufweist und die Abstandshalter die erste und zweite Oberfläche verbinden, und Aushärten der Abstandshalter, wobei das Polymermaterial der Abstandshalter nach dem Aushärten elastisch verformbar ist und die Abstandshalter in einem mechanisch unbelasteten Zustand die erste und die zweite Oberfläche in einem Abstand A beabstanden.

In einer Weiterbildung des vorstehenden Verfahrens erfolgt jeweils zunächst das Aufbringen einer oder mehrerer elastisch verformbarer, elektrisch leitender erster/zweiter Leitungen auf oder an der ersten/zweiten Oberfläche und anschließend das Aufbringen der zweiten/dritten Abstandshalter aus einem aushärtbaren Polymermaterial in einer zweiten/dritten Anordnung auf der ersten/zweiten Oberfläche, wobei die zweiten/dritten Abstandshalter auch teilweise auf die bereits vorhandenen ersten/zweiten Leitungen aufgebracht werden können. Anmerkung: Die ersten Abstandshalter und die Verbindung aus zweiten und dritten Abstandshaltern werden nachfolgend vereinfachend als jeweils auch als "Abstandshalter" bezeichnet.

Die Abstandshalter werden bevorzugt vereinzelt, d.h. sich nicht berührend, aufgebracht. Weiterhin werden die ersten und zweiten Leitungen bevorzugt derart auf die jeweiligen Oberflächen aufgebracht, dass sie im fertig hergestellten Sensorelement zwischen den Abstandshaltern angeordnet sind. Dies trifft insbesondere für die Kreuzungsstellen zu.

Ergänzend werden vor dem entsprechenden Fügen der ersten und zweiten Schicht, auf die erste bzw. zweite Oberfläche die elastisch verformbare, elektrisch leitende erste und zweite Leitung aufgebracht, wobei sich die ersten und zweiten Leitungen an Kreuzungsstellen kreuzen. Je nach Wahl der Dicke der Leitungen und des Abstandes kann eingestellt werden, ob die ersten und zweiten Leitungen im mechanisch unbelasteten Zustand an den Kreuzungsstelle voneinander beabstandet sind oder sie sich an den Kreuzungsstellen berühren. Die ersten und die zweiten Leitungen bestehen bevorzugt aus einem elektrisch leitenden Polymermaterial, wie cis-Polyacetylen (PA), trans-Polyacetylen (PA) oder Poly-Para-Phenylen (PPP). Alternativ können die ersten und zweiten Leitungen jeweils aus einem nichtleitenden und elastisch verformbaren Leitungskörper mit darin eingelagerten elektrisch leitenden Partikeln bestehen. Die ersten und zweiten Leitung liegen bevorzugt bereits vor deren Aufbringen auf die ersten und zweiten Oberflächen in einem ausgehärteten Zustand, d.h. vernetzt, vulkanisiert oder ausgehärtet vor. Alternativ können die ersten und zweiten Leitungen, analog zu den Abstandshaltern als ein aushärtbares elastisches Material aufgebracht und zeitlich vor dem Aufbringen der Abstandshalter oder gemeinsam mit den Abstandshaltern ausgehärtet werden.

Das aushärtbare Polymermaterial der Abstandshalter ist bevorzugt ein Thermoplastmaterial oder ein Silikonmaterial, wobei unter dem Begriff "aushärtbar" vorliegend verstanden wird, dass das elastische Material bzw. das Polymermaterial der Abstandshalter im Zeitpunkt des Aufbringens auf die erste bzw. zweite Oberfläche noch nicht vollständig vernetzt, bzw. nicht vollständig vulkanisiert bzw. nicht vollständig ausgehärtet ist.

Das Aufbringen der Abstandshalter bzw. des aushärtbaren elastischen Materials der elektrischen Leitungen erfolgt bevorzugt mittels eines Druck-, Press-, Gieß-, Spritzguss-, Rakel- oder Kalandrierverfahrens. Die erste Oberfläche bzw. die zweite Oberfläche und die darauf aufgebrachten Abstandshalter verbinden sich nach deren Aufbringen, ggf. ist hierfür eine chemische oder physikalische Vorbehandlung, bspw. Erwärmen der ersten/zweiten Oberfläche erforderlich.

Zur Einstellung eines vorbestimmten Verformungsverhaltens des Sensorelements können je nach Anforderung für die erste, die zweite Schicht und die Abstandshalter gleiche oder unterschiedliche elastische Materialien verwendet werden. Das richtungsabhängige (anisotrope) Verformungsverhalten des Sensorelements lässt sich weiterhin durch die Wahl der Anordnung der Abstandshalter einstellen. Vorzugsweise weist die Anordnung der Abstandshalter eine trapezförmige, ringförmige, elliptische oder rechteckige, insbesondere quadratische Geometrie auf. Natürlich sind je nach Anforderungen beliebige weitere Anordnungen realisierbar. Die Abstandshalter selbst können beliebige Formen annehmen, die sich im Wesentlichen aus den Anforderungen für das Sensorelement ergeben. So können die Abstandshalter zylinderförmig, wandartig, kubisch oder als einschaliger Hyperboloid etc. ausgeführt sein.

Nachdem die Abstandshalter mit der ersten und der zweiten Oberfläche verbunden sind, werden die erste und die zweite Schicht auseinander bewegt, sodass die erste und die zweite Oberfläche einen vorgegebenen Abstand voneinander aufweisen und die Abstandshalter die erste und zweite Oberfläche verbinden. Der vorgegebene Abstand ist in jedem Fall so zu wählen, dass die Abstandshalter die erste und zweite Oberfläche weiterhin verbinden, d.h. nicht reißen oder sich von einer oder beiden der ersten und zweiten Oberflächen lösen. Durch das Auseinenderbewegen der ersten und zweiten Schicht werden die Abstandshalter in ihren Außenformen verändert. Typischerweise stellt sich hierbei eine zylinderförmige oder einschalig hyperboloide Außenkontur der Abstandshalter ein. Weiterhin wird durch das Auseinanderbewegen die Größe der zwischen den ersten Abstandhaltern und den ersten und zweiten Oberfläche ausgebildeten Zwischenräume eingestellt. Über das Einstellen der Form der Abstandshalter und der Größe der Zwischenräume kann wiederum das richtungsabhängige (anisotrope) Verformungsverhalten des Folienelements vorgegeben bzw. eingestellt werden.

Nach dem Auseinanderbewegen erfolgt das Aushärten der Abstandshalter, wobei das Polymermaterial der Abstandshalter nach dem Aushärten elastisch verformbar bleibt und die Abstandshalter in einem mechanisch unbelasteten Zustand die erste und die zweite Oberfläche in einem Abstand A beabstanden. Der Begriff "Aushärten" wird vorliegend im Sinne von "vernetzen", "vulkanisieren", "abkühlen" oder "aushärten", je nach verwendetem Polymermaterial verwendet. Nach dem Aushärten ist das Polymermaterial der Abstandshalter elastisch verformbar und im Wesentlichen formstabil.

Bei einem weiteren Ausführungsbeispiel des Instruments ist vorgesehen, dass der Sensorkopf beziehungsweise der taktile Sensor eine Formgebung aufweisen. Vorteilhaft kann die Formgebung die vergleichsweise große taktil sensitive Oberfläche bereitstellen. Aufgrund elastischer Eigenschaften des taktilen Sensors kann die Formgebung vorübergehend während des Einführens beziehungsweise Durchführens oder Herausnehmens durch den Zugangskanal verloren gehen, so dass der taktile Sensor durch den Zugangskanal hindurchpasst.

Bei einem weiteren Ausführungsbeispiel des Instruments ist vorgesehen, dass die Formgebung ein von einer Richtung eines mittels der Fluidstromquelle erzeugbaren Fluidstroms abhängiges Entfalten oder Einfalten des Sensorelements und/oder ein Einrollen und Ausrollen des Sensorelements des taktilen Sensors bewirkt. Vorteilhaft kann das Sensorelement zum Herstellen beziehungsweise wieder Zurückbilden der taktil sensitiven Oberfläche entfaltet beziehungsweise eingefaltet und/oder ausgerollt beziehungsweise eingerollt werden. Dabei ist es denkbar, dass aufgrund der Formgebung und elastischen Eigenschaften das Einrollen beziehungsweise Ausrollen mittels Rückstellkräften selbsttätig erfolgt. Alternativ und/oder zusätzlich kann das Einrollen und Ausrollen beziehungsweise Entfalten und Einfalten mittels des Fluidstroms erfolgen.

Bei einem weiteren Ausführungsbeispiel des Instruments ist vorgesehen, dass die Behandlungsvorrichtung ein dem taktilen Sensor nachgeschaltetes taktiles Display aufweist, mittels dem während des Vorgangs von dem taktilen Sensor aufgenommene taktile Stimuli anzeigbar sind. Vorteilhaft kann dadurch eine direkte taktile Überwachung durch eine Person, beispielsweise eine Palpation von Gewebe und/oder eine direkte Überwachung von Anpresskräften des Instruments an Organe erfolgen.

Die Aufgabe ist außerdem bei einem Verfahren zum Betreiben eines minimalinvasiven Instruments, insbesondere eines vorab beschriebenen minimalinvasiven Instruments, gelöst. Es sind ein Einführen eines ein taktiles Sensorelement aufweisenden distalen Endes des Instruments durch einen einen Zugangskanalquerschnitt aufweisenden Zugangskanal und danach ein Bereitstellen einer taktil sensitiven Oberfläche des taktilen Sensorelements mit einer flächigen Ausdehnung, die ein Maß des Zugangskanalquerschnitts übersteigt, vorgesehen. Vorteilhaft kann nach dem Einführen die vergleichsweise große taktil sensitive Oberfläche des taktilen Sensorelements bereitgestellt werden, wobei vorteilhaft von einer vergleichsweise großen Fläche taktile Stimuli aufgenommen werden können.

Die Aufgabe ist außerdem bei einem Verfahren zum Betreiben eines minimalinvasiven Instruments, insbesondere eines vorab beschriebenen minimalinvasiven Instruments, mit Einführen eines ein taktiles Sensorelement aufweisenden distalen Endes des Instruments durch einen einen Zugangsquerschnitt aufweisenden Zugangskanal , und danach Bereitstellen einer taktil sensitiven Oberfläche des taktilen Sensorelements mit einer flächigen Ausdehnung, die ein Maß des Zugangsquerschnitts übersteigt, wobei das Sensorelement ein Folienelement mit einer ersten Schicht und einer mittels Abstandshaltern von der ersten Schicht beabstandeten zweiten Schicht aufweist und zwischen den Schichten des Folienelements angeordnete, dehnungssensitive, polymerbasierte Widerstandselemente zum Aufnehmen von taktilen Stimuli aufweist, Aufnehmen der taktilen Stimuli, wobei die taktilen Stimuli Dehnungen der polymerbasierten Widerstandselemente und die Dehnungen der polymerbasierten Widerstandselemente eine Änderung einer elektrischen Leitfähigkeit der polymerbasierten Widerstandselemente bewirken und Wandeln der Änderung der elektrischen Leitfähigkeit der polymerbasierten Widerstandselemente in ein elektrisches Widerstandssignal mittels einer Beschaltung gelöst. Das Widerstandssignal ist vorteilhaft ein Maß für die aufgenommenen taktilen Stimuli beziehungsweise beschreibt diese. Das Verfahren kann mittels eines vorab beschriebenen minimalinvasiven Instruments durchgeführt werden. Es ergeben sich die vorab beschriebenen Vorteile.

Die Aufgabe ist außerdem bei einem Verfahren zum Betreiben eines minimalinvasiven Instruments, insbesondere eines vorab beschriebenen minimalinvasiven Instruments, mit Vergrößern des Sensorkopfs, Aufnehmen der taktilen Stimuli, wobei die taktilen Stimuli Dehnungen der polymerbasierten Widerstandselemente und die Dehnungen der polymerbasierten Widerstandselemente eine Änderung einer elektrischen Leitfähigkeit der polymerbasierten Widerstandselemente bewirken, Wandeln der Änderung der elektrischen Leitfähigkeit der polymerbasierten Widerstandselemente in ein elektrisches Widerstandssignal mittels einer Beschaltung gelöst. Das Widerstandssignal ist vorteilhaft ein Maß für die aufgenommenen taktilen Stimuli beziehungsweise beschreibt diese. Das Verfahren kann mittels eines vorab beschriebenen minimalinvasiven Instruments durchgeführt werden. Es ergeben sich die vorab beschriebenen Vorteile.

Bei einem Verfahren ist ein Befüllen eines von dem Sensorelement zumindest teilweise fluiddicht umschlossenen Hohlraums mit einem Fluid zum Vergrößern eines Sensorquerschnitts des distalen Endes und Bereitstellen der taktil sensitiven Oberfläche vorgesehen. Vorteilhaft kann mittels des Fluids ein hydrostatischer Druck innerhalb des Hohlraums aufgebaut werden, so dass das Sensorelement eine gewünschte Form und Größe zum Bereitstellen der taktil sensitiven Oberfläche einnimmt.

Bei einem Verfahren sind ein Entleeren des Hohlraums und dabei Verkleinern des Zugangskanalquerschnitts und danach Entnehmen des distalen Endes mit dem Sensorelement durch den Zugangskanal vorgesehen. Vorteilhaft kann das Sensorelement nach dem Vorgang wieder verkleinert und über den Zugangskanal entnommen werden. Das Verkleinern erfolgt insbesondere mittels Rückstellkräften, hervorgerufen durch elastische Eigenschaften des Sensorelements. Alternativ oder zusätzlich kann auch ein Unterdruck in dem Hohlraum erzeugt werden.

Bei einem Verfahren sind ein Befüllen des Hohlraums mittels eines nachströmenden Fluids und/oder mittels einer Fluidstromquelle und/ oder ein Entleeren des Hohlraums mittels eines Ausströmens des Fluids und/oder mittels der Fluidstromquelle vorgesehen. Vorteilhaft kann mittels der Fluidstromquelle ein hydrostatischer Druck innerhalb des Hohlraums aufgebaut oder abgebaut werden. Alternativ und/oder zusätzlich kann eine Bewegung des Fluids aufgrund von Rückstellkräften einer Formgebung beziehungsweise aufgrund der elastischen Eigenschaften des Sensorelements erfolgen. Unter einem nachströmenden Fluid kann beispielsweise ein unter Druck stehendes und aus einer geöffneten Absperrvorrichtung ausströmendes Fluid verstanden werden, beispielsweise ein aus einem Druckgasanschluss ausströmendes Gas oder aus einem Wasserhahn ausströmendes Wasser. Zum Entleeren kann die Fluidstromquelle einen von dem Hohlraum abgesaugten Fluidstrom erzeugen, also als Saugpumpe betrieben werden.

Bei einem Verfahren ist ein Aufspannen des taktilen Sensorelements zum Bereitstellen der taktil sensitiven Oberfläche vorgesehen. Vorteilhaft kann das taktile Sensorelement zum Vergrößern beziehungsweise Bereitstellen der taktil sensitiven Oberfläche aufgespannt werden. Dabei ist es vorteilhaft möglich, dass sich das taktile Sensorelement vor dem Aufspannen in einem zusammengefalteten Zustand befindet, so dass dieses durch den Zugangskanal geschoben werden kann.

Weitere Vorteile, Merkmale und Einzelheiten ergeben sich aus der nachfolgenden Beschreibung, in der - gegebenenfalls unter Bezug auf die Zeichnung - zumindest ein Ausführungsbeispiel im Einzelnen beschrieben ist. Gleiche, ähnliche und/oder funktionsgleiche Teile sind mit gleichen Bezugszeichen versehen.

Es zeigen:

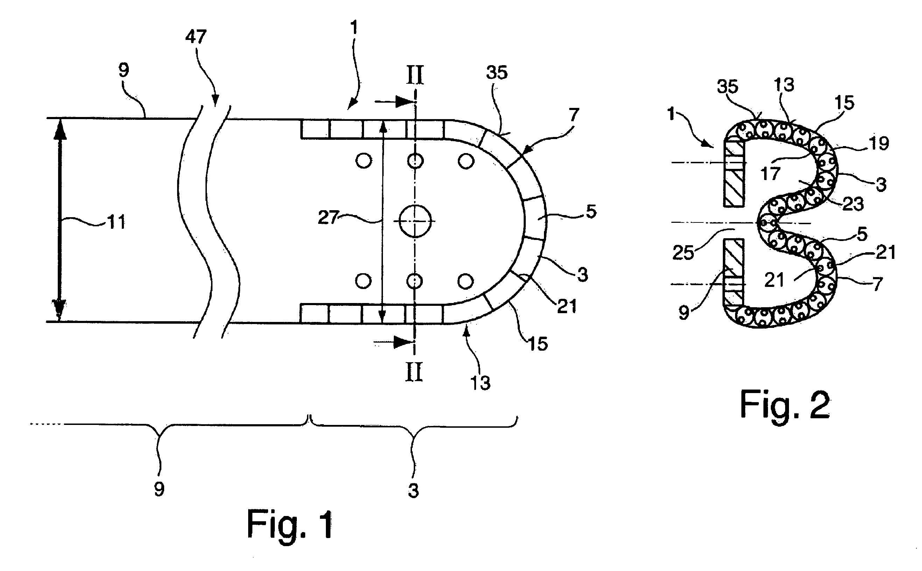

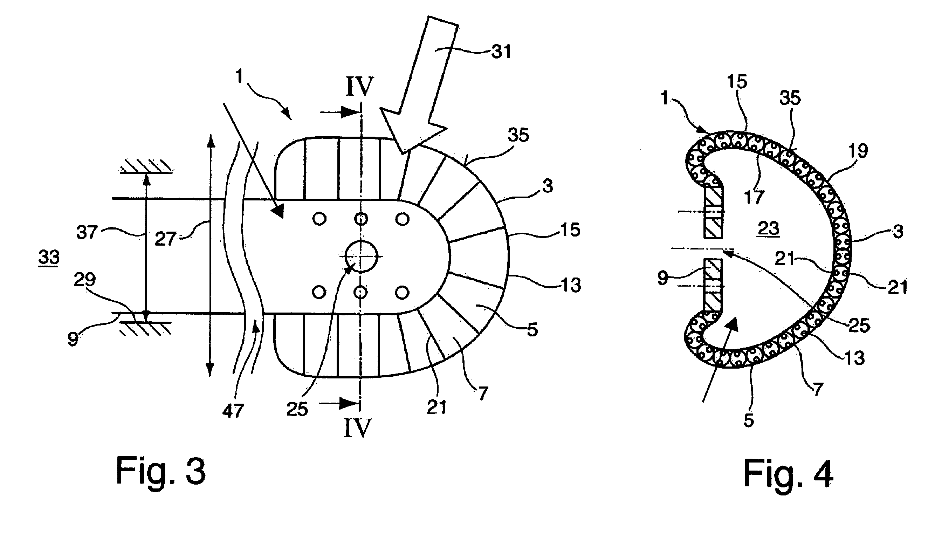

Fig. 1 eine Draufsicht auf ein distales Ende eines minimalinvasiven Instruments mit einem taktil sensitiven Sensorelement eines taktilen Sensors, wobei ein Sensorquerschnitt des distalen Endes minimal ist; Fig. 2 einen Querschnitt entlang der Linie II-II des in Fig. 1 dargestellten distalen Endes; Fig. 3 die in Fig. 1 dargestellte Draufsicht des distalen Endes, wobei das Sensorelement in einem bereitgestellten Zustand einer taktil sensitiven Oberfläche dargestellt ist, wobei der Sensorquerschnitt vergrößert ist;

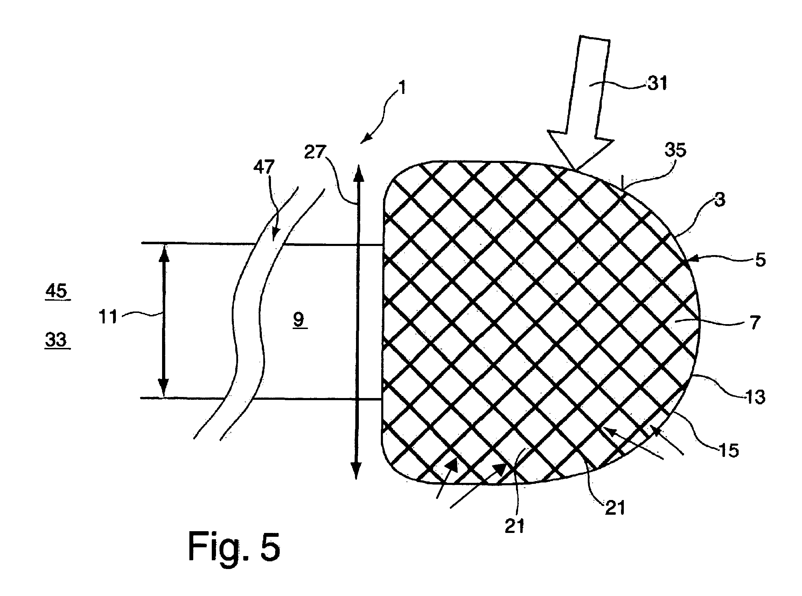

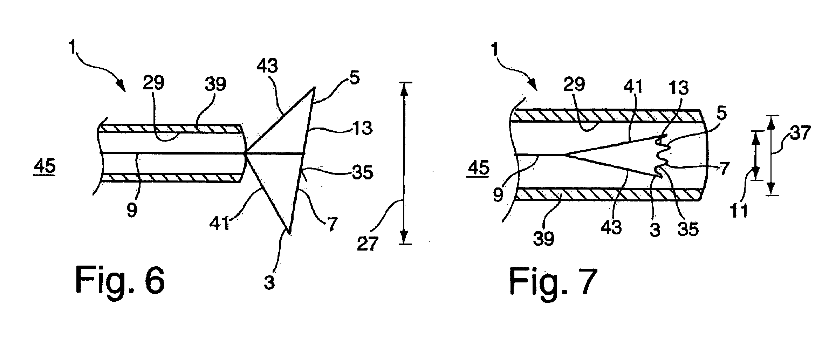

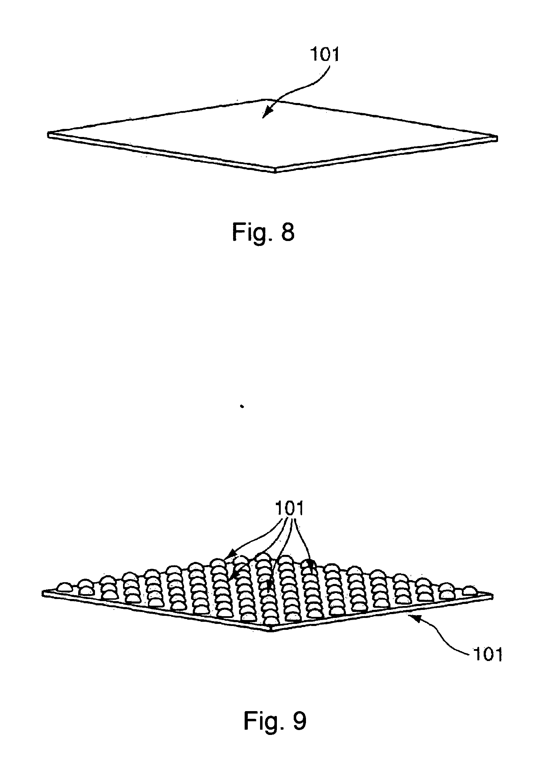

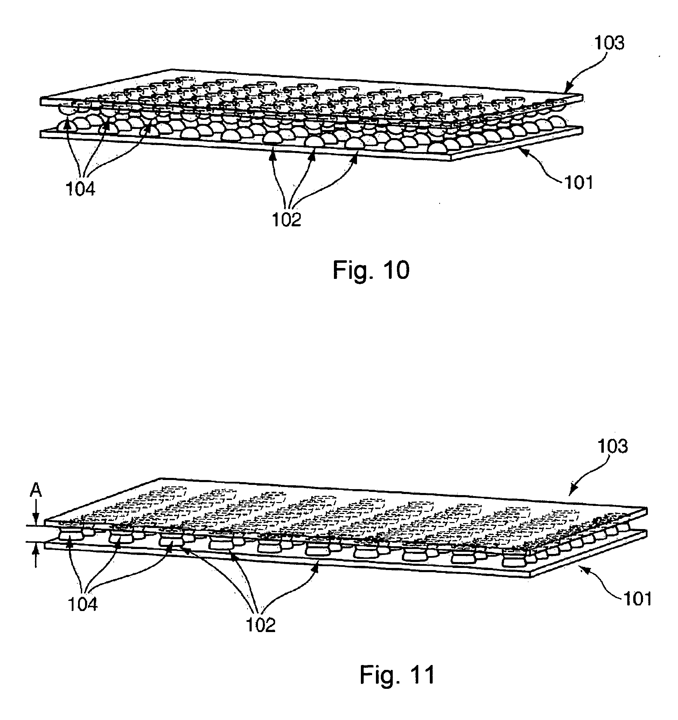

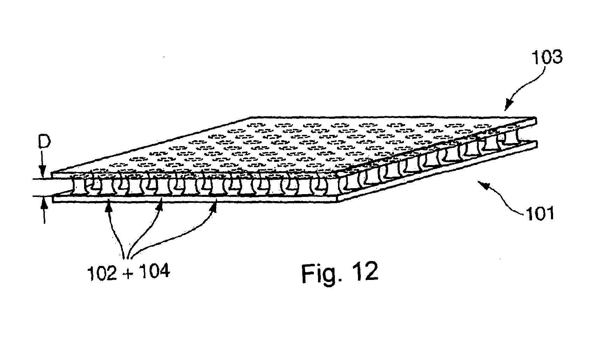

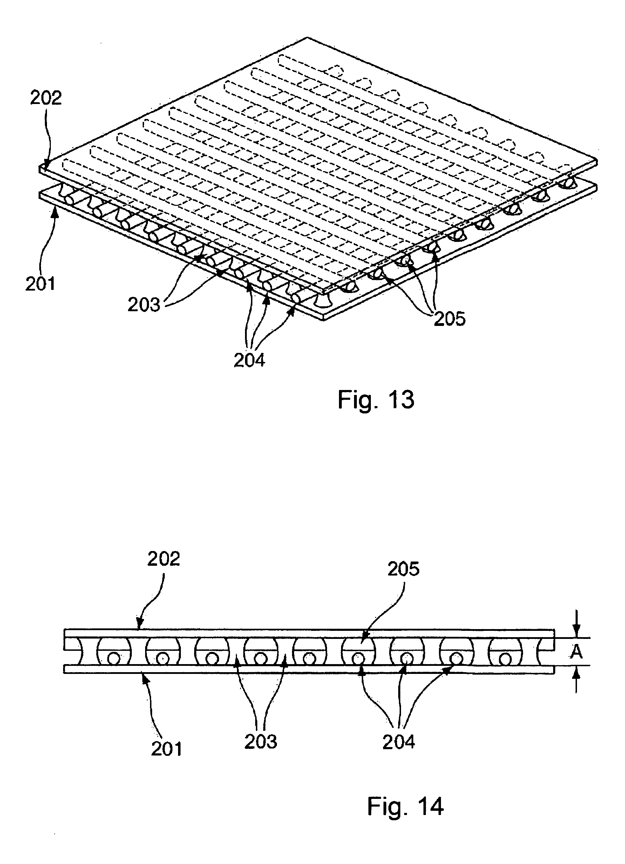

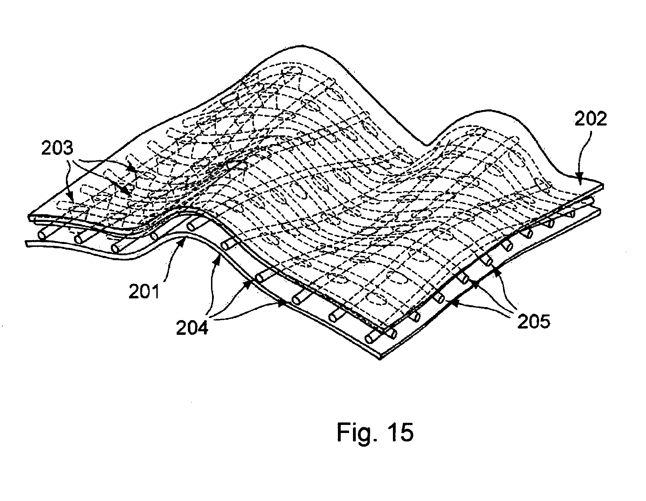

Fig. 4 einen Querschnitt entlang der Linien IV-IV des in Fig. 3 dargestellten distalen Endes; Fig. 5 eine Untenansicht des in Fig. 3 gezeigten distalen Endes auf die taktil sensitive Oberfläche; Fig. 6 einen Längsschnitt eines distalen Endes eines weiteren minimalinvasiven Instrument, wobei eine taktil sensitive Oberfläche mittels einer Spannvorrichtung bereitgestellt ist; Fig. 7 die in Fig. 6 gezeigte Schnittansicht des distalen Endes, wobei sich im Unterschied das distale Ende zusammen mit der taktil sensitiven Oberfläche in einem zusammengefalteten Zustand innerhalb eines Zugangskanals befindet; Fig. 8 eine bereitgestellte erste Schicht eines für das minimalinvasive Instrument verwendeten Folienelements; Fig. 9 auf die erste Oberfläche der ersten Schicht aufgebrachte zweite Abstandshalter des Folienelements; Fig. 10 eine ausgerichtete erste und zweite Schicht des Folienelements; Fig. 11 Zustand beim Fügen der zweiten und dritten Abstandshalter des Folienelements; Fig. 12 Zustand nach dem Auseinanderbewegen der ersten und zweiten Schicht bis eine gewünschte Gesamtdicke des Folienelements erreicht ist; Fig. 13 eine schematische Darstellung eines vorteilhaften Sensorelements verwendbar für das in den Figuren 1 bis 7 dargestellte minimalinvasive Instrument in Schrägansicht; Fig. 14 eine schematische Darstellung des Sensorelements von Fig. 13 in Seitenansicht; und Fig. 15 eine schematische Darstellung des Sensorelements von Fig. 13 bzw. Fig. 14 für eine Anwendung auf einer dreidimensional geformten Oberfläche.

Fig. 1 zeigt eine Draufsicht auf ein nur teilweise dargestelltes minimalinvasives Instrument 1. Dargestellt ist ein vergößerbarer Sensorkopf eines distales Ende 3 des minimalinvasiven Instruments 1. Das distale Ende 3 beziehungsweise der Sensorkopf weisen einen taktilen Sensor 5 auf. Mittels des taktilen Sensors 5 sind taktile Stimuli erfassbar. Dazu weist der taktile Sensor 5 ein Sensorelement 7 sowie eine dem Sensorelement 7 nachgeschaltete Auswerteeinheit 49 auf.

Das Instrument 1 weist einen Schaft 9 auf, der das distale Ende 3 aufweist. Der Schaft 9 beziehungsweise das distale Ende 3 des Schafts 9 kann durch einen nicht näher dargestellten Zugangskanal hindurchgeführt werden. Bei dem Zugangskanal kann es sich beispielsweise um eine natürliche Körperöffnung und/oder um einen Trokar, der eine künstliche Körperöffnung offen hält, handeln.

Der Schaft 9 weist einen Instrumentenquerschnitt und am distalen Ende 3 einen verkleinerten Endquerschnitt auf, der durch ein erstes Maß gekennzeichnet ist. Das erste Maß ist in Fig. 1 mittels eines ersten Doppelpfeils 11 und eines zweiten Doppelpfeils 27 symbolisiert. Der Instrumentenquerschnitt und/oder der Sensorquerschnitt können beispielsweise durch einen Durchmesser, eine Breite, eine Höhe, eine Fläche und/oder ein ähnliches Maß gekennzeichnet sein. Der Sensorquerschnitt des distalen Endes 3 und der Instrumentenquerschnitt des Schafts 9 sind so dimensioniert, dass sie durch den Zugangskanal, der in Fig. 1 nicht näher dargestellt ist, hindurchführbar, also kleiner als der Zugangskanalquerschnitt sind.

In Figur 1 ist die Auswerteeinheit 49 zu erkennen, die an das Sensorelement 7 beziehungsweise an die Widerstandselemente 21 des Sensorelements 7 angeschlossen ist. Mittels der Beschaltung kann vorteilhaft eine Änderung einer Leitfähigkeit der polymerbasierten Widerstandselemente 21 in ein elektrisches Widerstandssignal gewandelt werden, beispielsweise einen Strom oder eine Spannung. Die Änderung der Leitfähigkeit rührt von einer Dehnung der Widerstandselemente 21 her, die wiederum durch den taktilen Stimulus verursachbar ist.

Fig. 2 zeigt einen Querschnitt entlang der Linie II-II des in Fig. 1 gezeigten distalen Endes 3 des Instruments 1.

Es ist zu erkennen, dass das Sensorelement 7 einen zusammengefalteten Zustand aufweist, wobei sich das Sensorelement 7 mäanderförmig zusammengefaltet an das distale Ende 3 des Instruments 1 anlegt.

Das Sensorelement 7 weist eine Formgebung mit elastischen Eigenschaften auf, so dass die mäanderförmige Faltung reversibel hergestellt, also eingefaltet und ausgefaltet werden kann, insbesondere durch Rückstellkräfte der Formgebung, insbesondere der Faltung.

Das Sensorelement 7 des taktilen Sensors 5 weist ein Folienelement 13 mit einer ersten Schicht 15 und einer zweiten Schicht 17 auf. Die Schichten 15 und 17 sind mittels Abstandhaltern 19 voneinander beabstandet. Zwischen den Schichten 15 und 17, also in einem zwischen diesen verbleibenden Zwischenraum sind piezo-resistive Widerstandselemente 21 angeordnet, mittels denen taktile Stimuli auflösbar sind. Die Widerstandselement 21 sind der nicht näher dargestellten Auswerteeinheit vorgeschaltet.

Dadurch entsteht innerhalb einer Außenfläche des Schafts 9 und innerhalb des Folienelements 13 des Sensorelements 7 des taktilen Sensors 5 ein Hohlraum 23. Die in Richtung einen Inneren des Hohlraums 23 zeigende Außenfläche des Schafts 9 und das Folienelement 13 des Sensorelements 7 des taktilen Sensors 5 umschließen den Hohlraum 23 fluiddicht. Dem Hohlraum 23 ist ein in diesen mündender Fluidpfad 25 zugeordnet. Der Schaft 9 beziehungsweise die Außenfläche des Schafts 9 weisen einen Durchbruch auf, wobei der Fluidpfad 25 durch den Durchbruch des Schafts 9 hindurchführt. Über den Fluidpfad 25 kann der fluiddichte Hohlraum 23 mit einem Fluid befüllt oder entleert werden. Bei dem Fluid kann es sich beispielsweise um eine Flüssigkeit, insbesondere Wasser, oder ein Gas, insbesondere Luft, handeln.

Die Fig. 3 und 4 zeigen im Wesentlichen dieselben Darstellungen wie die Fig. 1 und 2, wobei im Unterschied der Hohlraum 23 über den Fluidpfad 25 mit dem Fluid befüllt ist. Es ist zu erkennen, dass sich dadurch das Folienelement 13 des Sensorelements 7 entfaltet beziehungsweise aufgrund wirkender hydrostatischer Kräfte ballonförmig aufspannt.

Aufgrund des gefüllten Hohlraums 23 nimmt das Folienelement 13 einen vergrößerten Sensorquerschnitt ein, wobei der Instrumentenquerschnitt unverändert ist. Der vergrößerte Sensorquerschnitt weist ein größeres Maß als der in Fig. 1 und 2 dargestellte Sensorquerschnitt auf. Das zweite Maß des vergrößerten Instrumentenquerschnitts ist in Fig. 3 mittels des zweiten Doppelpfeils 27 symbolisiert. Es ist zu erkennen, dass in den Darstellungen gemäß der Fig. 3 und 4 der vergrößerte Sensorquerschnitt gegenüber dem in den Fig. 1 und 2 dargestellten Sensorquerschnitt derartig größer ist, dass das distale Ende 3 nicht mehr kollisionsfrei durch einen in Fig. 3 lediglich mittels des Bezugszeichens 29 und zwei Strichen angedeuteten, teilweise dargestellten Zugangskanal 29 führbar ist. Im bereitgestellten Zustand sind also der Sensorquerschnitt und damit auch eine taktil sensitive Oberfläche 35 des taktilen Sensors größer als der Zugangskanalquerschnitt.

Eine Formgebung des gemäß den Fig. 3 und 4 entfalteten Sensorelements 7 ist frei wählbar.

In Fig. 3 ist mittels eines Pfeils ein mechanischer Stimulus symbolisiert, der auf das Folienelement 13 des Sensorelements 7 des taktilen Sensors 5 wirkt. Der mechanische Stimulus gemäß des Pfeils 31 bewirkt eine Verformung des Folienelements 13 und damit eines der entsprechend darunter angeordneten Widerstandselemente 21, was wiederum mittels der nicht näher dargestellten Auswerteeinheit aufgelöst werden kann.

Fig. 5 zeigt das distale Ende 3 des Instruments 1 in einer Untenansicht, wobei der Hohlraum 23 analog der Darstellungen der Fig. 3 und 4 mit dem Fluid befüllt ist.

Zum Befüllen des Hohlraums 23 mit dem nicht näher dargestellten Fluid kann der Fluidpfad 25 an eine in Fig. 4 lediglich mittels des Bezugszeichens 33 symbolisierte Fluidstromquelle 33 angeschlossen werden. Mittels der Fluidstromquelle 33, die insbesondere als bidirektionale Pumpe ausgelegt sein kann, kann der Hohlraum 23 wahlweise mit dem Fluid befüllt oder entleert werden.

In Fig. 5 ist die taktil sensitive Oberfläche 35 des Sensorelements 7 des taktilen Sensors 5 zu erkennen. Mittels des Pfeils 31 ist das Einwirken des mechanischen Stimulus auf die taktil sensitive Oberfläche 35 zu erkennen. In einem Bereich des taktilen Stimulus weist das Sensorelement 7 des taktilen Sensors 5 eine erste taktile Auflösung auf. In einem benachbarten Bereich kann das das Sensorelement 7 des taktilen Sensors 5 vorteilhaft eine davon abweichende zweite taktile Auflösung aufweisen. Insbesondere kann besonders vorteilhaft die taktile Auflösung richtungsabhängig sein. Dadurch können beispielsweise bei einem Überstreichen von Gewebe in unterschiedliche Richtungen unterschiedliche Sensorsignale gewonnen werden, die genauere Rückschlüsse auf die Beschaffenheit des Gewebes zulassen.

Mittels des zweiten Doppelpfeils 27 ist symbolisiert, dass auch die taktil sensitive Oberfläche 35 eine flächige Ausdehnung aufweist, die das mittels des zweiten Doppelpfeils 27 in Fig. 1 dargestellte erste Maß des verkleinerten Sensorquerschnitts übersteigt. Vorteilhaft kann also nach dem Durchführen des distalen Endes 3 des Instruments 1 durch den Zugangskanal 29 eine im Vergleich zu dem Zugangskanalquerschnitt des Zugangskanals 29 vergleichsweise große taktil sensitive Oberfläche 35 bereitgestellt werden. Ein zweites Maß des Zugangskanalquerschnitts des Zugangskanals 29 ist in Fig. 3 mittels eines dritten Doppelpfeils 37 symbolisiert. Dabei kann es sich beispielsweise um einen Durchmesser des Zugangskanals 29 handeln, jedoch auch um ein beliebiges anderes Maß, beispielsweise eine Querschnittsfläche, eine Breite, eine Höhe und/oder Ähnliches.

Die Fig. 6 und 7 zeigen ein weiteres Ausführungsbeispiel eines minimalinvasiven Instruments 1 zusammen mit einem Zugangskanal 29, wobei in Fig. 6 das Instrument 1 ein einem aufgefalteten Zustand mit einer bereitgestellten und aufgespannten taktil sensitiven Oberfläche 35 des Folienelements 13 des Sensorelements 7 des taktilen Sensors 5 und in Fig. 7 das Instrument 1 innerhalb des Zugangskanals 29 in einem zusammengefalteten Zustand mit der zusammengefalteten taktil sensitiven Oberfläche 35 des Folienelements 13 des Sensorelements 7 des taktilen Sensors 5 dargestellt ist. Der Zugangskanal 29 kann beispielsweise mittels eines Trokars 39 ausgebildet sein.

Zum Aufspannen der taktil sensitiven Oberfläche 35, also zum Aufspannen des Folienelements 13 des Sensorelements 7 des taktilen Sensors 5 des distalen Endes 3, weist das distale Ende 3 des Instruments 1 eine Spannvorrichtung 41 auf. Die Spannvorrichtung 41 kann beispielsweise dem Folienelement 13 zugeordnete, schwenkbare Spannstäbe 43 aufweisen, die mit dem Folienelement verbunden sind und dieses durch ein Auseinanderschwenken aufspannen, und umgekehrt.

Vorteilhaft kann mittels des Instruments 1 ein haptisches Feedback während des Vorgangs erzeugt werden. Dies kann insbesondere während eines minimalinvasiven Eingriffs vorteilhaft erfolgen.

Alternativ und/oder zusätzlich ist es denkbar, dem taktilen Sensor 5 ein in den Fig. 6 und 7 lediglich mittels des Bezugszeichens 45 angedeutetes taktiles Display 45 nachzuschalten. Dabei können mittels des taktilen Sensors 5 aufgenommene mechanische Stimuli vorteilhaft mittels des taktilen Displays 45 dargestellt werden, so dass ein haptisches Feedback trotz einer indirekten Durchführung des Vorgangs mittels des Instruments 1 erzeugbar ist. Dabei kann eine flächige Kraft- und/oder Druckverteilung, die auf die taktil sensitive Oberfläche 35 des Sensorelements 7 wirkt, mittels des taktilen Displays 45 dargestellt beziehungsweise übertragen werden, beispielsweise auf Finger eines Bedieners des minimalinvasiven Instruments 1. Alternativ und/oder zusätzlich kann vorteilhaft eine sogenannte Kraftrückkopplung während des Vorgangs erfolgen.

Vorteilhaft weist der taktile Sensor 5 flexible Eigenschaften auf, so dass vorteilhaft während des Vorgangs eine Palpation und/oder beispielsweise während eines Anhebens eines Organs eine dabei auftretende Flächenpressung überwachbar ist, insbesondere um eine Durchblutung des Organs abzuschätzen und/oder zu überwachen beziehungsweise die Anpresskraft so einzustellen, dass diese nicht gefährdend ist.

Vorteilhaft kann das distale Ende 3 des Instruments 1 durch den Zugangskanal 29, insbesondere den Zugangskanal 29 des Trokars 39, hindurchgeführt werden, wobei vorteilhaft dadurch der größere Sensorquerschnitt beziehungsweise ein entsprechender zweiter Durchmesser nicht limitiert ist. Vielmehr kann vorteilhaft die taktil sensitive Oberfläche 35 nach dem Durchführen des distalen Endes 3 des Instruments 1 mittels Befüllen des Hohlraums 23 und/oder Spannen der Spannvorrichtung 31 vergrößert werden, so dass eine vergleichsweise große taktil sensitive Oberfläche 35 zum Untersuchen von größeren Flächen, beispielsweise zur Palpation, zur Verfügung steht.

Der taktile Sensor 5 beziehungsweise das Sensorelement 7 kann ein polymerbasiertes elastisches Material aufweisen.

Der Hohlraum 23 kann mittels der Fluidstromquelle 33 mit einem hydrostatischen, insbesondere hydraulischen und/oder pneumatischen Druck beaufschlagt werden, um dadurch das Sensorelement 7 beziehungsweise die taktil sensitive Oberfläche 35 des taktilen Sensors 5 zu entfalten. Vorteilhaft entfällt eine Limitierung der taktil sensitiven Oberfläche 35 durch den Zugangskanalquerschnitt des Zugangskanals 29, insbesondere des Trokars 39.

Alternativ und/oder zusätzlich kann durch eine vorteilhafte Wahl einer Geometrie des Hohlkörpers 23 eine Vielzahl geformter taktiler Sensoren realisiert werden, insbesondere um diese aufgabenspezifisch an den Vorgang anzupassen. Vorteilhaft ergibt sich ein hochflexibler, formveränderbarer taktiler Sensor 5, der vorteilhaft zusammen mit dem minimalinvasiven Instrument 1 eingesetzt werden kann.

Alternativ und/oder zusätzlich ist es denkbar, insbesondere mittels des taktilen Displays 45 eine simultane Palpation wesentlich größerer Flächen und damit eine sicherere Erkennung kleinerer und/oder größerer Verhärtungen in einem zu untersuchenden Weichgewebe zu ermöglichen.

Das Sensorelement 7 beziehungsweise die taktil sensitive Oberfläche 35 des Sensorelements 7 weist eine Sensormatrix auf, die von dem dreidimensionalen Hohlraum 23 definiert wird. Vorteilhaft kann die Sensormatrix beziehungsweise die taktil sensitive Oberfläche 35 in einer dreidimensionalen Form räumlich entsprechend des Hohlraums 23 verwendet werden.

Erfindungsgemäß ist eine elektromechanische Schnittstelle 47 vorhanden, mittels der das distale Ende 3 zusammen mit dem taktilen Sensor 5 und/oder der taktile Sensor 5 von dem übrigen minimalinvasiven Instrument 1 lösbar verbindbar ist.

Alternativ und/oder zusätzlich ist es denkbar, den Hohlraum 23 und/oder das Sensorelement 7 so zu gestalten, dass dieses eingerollt und ausgerollt und/oder zusammengefaltet und auseinandergefaltet werden kann.

Fig. 8 zeigt einen ersten Verfahrensschritt in dem eine erste Schicht 101 mit einer ersten Oberfläche aus einem elastisch verformbaren Material bereitgestellt wird. Die erste Schicht 101, vorliegend aus einem Silikonmaterial, kann in einem Press-, Gieß, Spinn-, Druck-, Tauch-, Sprüh- oder Kalandrierverfahren hergestellt sein. Die erste Schicht 101 kann als zweidimensionale ebene oder dreidimensional im Raum geformte Schicht ausgebildet sein. Die Schichtdicke der ersten und/oder der zweiten Schicht 103 können je nach Anwendung mehrere Millimeter aber auch Mikrometer bspw. < 500 µm, < 300 µm, < 200 µm, < 150 µm, < 100 µm, < 75 µm, < 50 µm betragen.

Fig. 9 zeigt die erste Schicht 101 nachdem auf die erste Oberfläche der ersten Schicht 101 vereinzelte zweite Abstandshalter 102 aus einem aushärtbaren Polymermaterial (vorliegend ein Silikonmaterial) in einer zweiten Anordnung aufgebracht worden sind. Deutlich zu erkennen ist, dass die Abstandshalter 102 einen Abstand voneinander aufweisen, der zumindest ihrem Durchmesser entspricht. Vorliegend wurden als zweite Abstandshalter Silikon-Bumps in einer quadratischen Anordnung, d.h. an den jeweiligen Ecken eines Quadrats, auf die erste Oberfläche aufgebracht.

Nicht dargestellt ist das Bereitstellen der zweiten Schicht 103 aus einem elastisch verformbaren Material mit einer zweiten Oberfläche, sowie das Aufbringen vereinzelter dritter Abstandshalter 104 aus einem aushärtbaren Polymermaterial, vorliegend ebenfalls ein Silikonmaterial, in einer zur zweiten Anordnung spiegelsymmetrischen dritten Anordnung auf der zweiten Oberfläche.

Fig. 10 zeigt das Ausrichten der ersten 101 und zweiten 103 Schicht, so dass sich die auf der ersten und auf der zweiten Oberfläche spiegelsymmetrisch aufgebrachten Anordnungen der zweiten 102 und dritten 104 Abstandshalter deckungsgleich gegenüberliegen.

Fig. 11 zeigt das Fügen der sich jeweils deckungsgleich gegenüberliegenden zweiten 102 und dritten 104 Abstandshalter an ihren jeweiligen freien Enden. Um den Fügevorgang im Fall von Thermoplastmaterialien zu beförden, wurden die freien Enden der zweiten 102 und dritten 104 Abstandshalter zuvor erwärmt. Alternativ oder zusätzlich kann das Fügen mittels mechanischer Vibration der ersten 101 und zweiten 103 Schicht befördert werden.

Nach Beendigung des Fügevorgangs sind die zweiten 102 und dritten Abstandshalter 104 miteinander verbunden, gleichermaßen sind die erste 101 und zweite 103 Schicht über die verbundenen Abstandshalter 102, 104 miteinander verbunden. Die verbundnen Abstandshalter 102, 104 liegen in diesem Verfahrensschritt als noch aushärtbares Polymermaterial, d.h. vorliegend als noch nicht vollständig vernetztes Silikonmaterial, vor. Dabei hat das Material zumindest noch eine geringe Fließfähigkeit.

Fig. 12 zeigt den Zustand des Folienelements nach dem Auseinanderbewegen der ersten 101 und zweiten 103 Schicht so, dass die erste und die zweite Oberfläche einen vorgegebenen Abstand voneinander aufweist und die gefügten Abstandshalter 102, 104 die erste und zweite Oberfläche verbinden. Deutlich zu erkennen ist, dass sich durch das Auseinanderbewegen die Außenkonturen der gefügten Abstandshalter 102, 104 einem einschaligen Hyperboloid angenähert haben, dessen Mittendurchmesser geringer ist, als der Fußdurchmesser jeweils an der ersten bzw. zweiten Oberfläche.

In einem letzten Verfahrensschritt (nicht dargestellt) erfolgt vorliegend noch ein Aushärten, d.h. vollständiges Vernetzen des Silikonmaterials der gefügten Abstandshalter 102, 104, wobei das Polymermaterial der gefügten Abstandshalter 102, 104 nach dem Aushärten elastisch verformbar bleibt und die Abstandshalter 102, 104 in einem mechanisch unbelasteten Zustand die erste und die zweite Oberfläche in einem Abstand A beabstanden. Das Folienelement weist dabei die Gesamtdicke D auf.

Fig. 13 zeigt eine schematische Darstellung eines Sensorelements des in den Figuren 1 bis 7 dargestellten minimalinvasiven Instruments 1 in Schrägsichtansicht. Dargestellt ist ein Sensorelement mit einer ersten Schicht 201 und einer zweiten Schicht 202. Je nach Einbaulage des Sensorelements, bildet entweder die erste Oberfläche 201 oder die zweite Oberfläche 202 die taktil snesitive Oberfläche 35 des in den Figuren 1 bis 7 dargestellten minimalinvasiven Instruments 1. Beide Schichten sind über Abstandshalter 203 miteinander verbunden und im Abstand A beabstandet voneinander angeordnet. Die Abstandshalter 203 sind vereinzelt, in quadratischer Gitterform angeordnet. In Zwischenräumen/Hohlräumen zwischen den Abstandshaltern 203 und der ersten 201 und zweiten 202 Schicht verlaufen erste 204 und zweite 205 elektrisch leitende, elastisch verformbare Leitungen. Die ersten Leitungen 204 verlaufen zueinander parallel. Ebenso die zweiten Leitungen 205, wobei die ersten 204 und zweiten 205 Leitungen sich kreuzend, vorliegend orthogonal zueinander, angeordnet sind. Weiterhin sind die ersten Leitungen 204 an der ersten Oberfläche der ersten Schicht 201 und die zweiten Leitungen 205 and der zweiten Oberfläche der zweiten Schicht 202 angeordnet. Die Kreuzungsstellen sind in Bereichen zwischen den Abstandshaltern 203 angeordnet. Im mechanisch unbelasteten Zustand kreuzen sich die ersten 204 und zweiten 205 Leitungen an den Kreuzungsstellen nicht-berührend. Die ersten 204 und zweiten 205 Leitungen können die vorab beschriebenen vorteilhaften Widerstandselemente 21 bilden.

Durch die Verwendung polymer-basierter erster 203 und zweiter 204 Leitungen(Leiterbahnen) ist das Sensorelement insgesamt elastisch verformbar ausgeführt. Es weist zwischen den Abstandshaltern 203 und der ersten Oberfläche der ersten Schicht 201 und der zweiten Oberfläche der zweiten Schicht 202 Zwischenräume/Hohlräume auf, die vorliegend luftgefüllt sind. Das Sensorelement kann bei entsprechender Wahl der verwendeten Materialien und Schichtdicken als hochflexibles, dünnes und elastisch dehnbares Sensorelement ausgeführt sein. Diese Eigenschaften ermöglichen es, dass das vorteilhafte Sensorelement auf stark gekrümmten Oberflächen, wie bspw. einem Greiffinger einer Roboterhand, einem medizinischen Instrument für minimalinvasive Eingriffe etc. aufgebracht werden kann, um dort den cutanen Tastsinn des Menschen nachzubilden und um so beispielsweise die Position, die Form, die stoffliche Konsistenz oder die Obersflächenstruktur berührter Objekte festzustellen.

Das Sensorelement ermöglicht die durch die Erfassung verschiedener Effekte eine kombinierte Druck- und Dehnungsmessung. Dabei werden folgende Effekte genutzt:
1. Elektrische Kapazitätsänderung
   An jedem Kreuzungspunkt der ersten 204 und zweiten 205 Leitungen wird zwischen der jeweiligen ersten Leitung 204 und der jeweiligen zweiten Leitung ein elektrischer Kondensator gebildet. Wird auf das Sensorelement ein taktiler Reiz aufgebracht, so wird das Sensorelement komprimiert und die ersten 204 und zweiten 205 Leitungen nähern sich einander an. Die elektrische Kapazität am jeweiligen Kreuzungspunkt ändert sich hierdurch. Dieser Effekt kann erfasst und ausgewertet werden. Durch den besonderen Aufbau des Sensorelements mit den Zwischenräumen und der über die Abstandshalter beabstandeten ersten 201 und zweiten 202 Schicht, müssen hierbei nur sehr kleine Materialvolumina verformt werden, damit sich die ersten 204 und zweiten 205 Leitungen einander annähern und so eine Kapazitätsänderung auftritt. Hierdurch wird für eine taktile Reizung des Sensorelements eine sehr hohe Empfindlichkeit erreicht, so dass bereits kleinste Berührungen erfasst werden können. Die Kapazitätsänderung an den Kreuzungspunkten kann zudem für die Identifizierung der mit Druck beaufschlagten Kreuzungspunkte und somit zur Erfassung der Druckverteilung auf das Sensorelement genutzt werden.
2. Elektrischer Übergangswiderstand
   Wird der taktile Reiz in seiner Intensität weiter erhöht, berühren sich die ersten 204 und zweiten 205 Leitungen an den betreffenden Kreuzungspunkten. Dadurch wir ein elektrischer Widerstand messbar. Bei weiterer Erhöhung der Reizintensität (Kraft- oder Druckerhöhung) verformen sich die ersten 204 und zweiten205 Leitungen an den betreffenden Kreuzungspunkten elastisch. Hierdurch verändert sich die Kontaktfläche zwischen den Leitungen an den jeweiligen Kreuzungspunkten und der elektrische Übergangswiderstand verändert sich. Diese Widerstandsänderung kann für jeden Kreuzungspunkt erfasst und ausgewertet werden.
3. Elektrischer Leitungswiderstand in den Leitungen
   Wird ein mechanisch weiches Material unter dem Sensorelement verwendet kann auch der in DE10 2007 020 131 beschriebene Effekt zur Bestimmung der mechanischen Dehnung erfasst werden. Hierbei wird die dehnungs-abhängige Änderung des elektrischen Leitungswiderstands in den Leitungen erfasst und ausgewertet. Werden die ersten 204 und zweiten 205 Leitungen beispielweise in einem Press- oder Druckverfahren hergestellt, so kann deren Querschnitt nahezu beliebig gewählt werden. Dies kann genutzt werden, um die Übertragungsfunktion des unter Punkt 2. beschriebenen Effekts zu linearisieren. Die verwendeten ersten 204 und zweiten 205 Leitungen in X- bzw. Y-Richtung werden vorliegend im mechanisch unbelasteten Zustand des Sensorelements durch die Abstandshalter 203 räumlich voneinander getrennt.

Fig. 14 zeigt eine schematische Darstellung des Sensorelements von Fig. 13 in Seitenansicht, in einem mechanisch unbelasteten Ruhezustand, d.h. einem Zustand in dem das Sensorelement vorliegt, sofern kein taktiler Reiz eingebracht wird. Deutlich zu erkennen sind die Abstandshalter 203, welche bedingt durch das Auseinanderbewegen der ersten 201 und zweiten 202 Schicht während des Herstellungsprozesses eine einschalige hyperboloide Außenkontur aufweisen. Die erste und die zweite Oberfläche sind mit einem Abstand A voneinander beabstandet. Weiterhin deutlich zu erkennen ist, dass die ersten 204 Leitungen und die zweiten Leitungen 205 sich an den Kreuzungsstellen nicht berühren.

Fig. 15 zeigt eine schematische Darstellung des Sensorelements von Fig. 13 bzw. Fig. 14 für eine Anwendung auf einer dreidimensional geformten Oberfläche. Durch die elastische Verformbarkeit des gesamten Sensorelements lassen sich auch dreidimensional geformte Oberflächen, wie bspw. ein Greifer, ein Roboterfinger, etc. mit dem flächigen taktilen Sensorelement ausrüsten.

### Bezugszeichenliste

- 1: minimalinvasives Instrument
- 3: distales Ende
- 5: taktiler Sensor
- 7: Sensorelement
- 9: Schaft
- 11: Doppelpfeil
- 13: Folienelement
- 15: erste Schicht
- 17: zweite Schicht
- 19: Abstandhalter
- 21: Widerstandselement
- 23: Hohlraum
- 25: Fluidpfad
- 27: Doppelpfeil
- 29: Zugangskanal
- 31: Pfeil
- 33: Fluidstromquelle
- 35: taktil sensitive Oberfläche
- 37: Doppelpfeil
- 39: Trokar
- 41: Spannvorrichtung
- 43: Spannstäbe
- 45: taktiles Display
- 47: elektromechanische Schnittstelle
- 49: Auswerteeinheit
- 101: erste Schicht
- 102: zweite Abstandshalter
- 103: zweite Schicht
- 104: dritte Abstandshalter
- A: Abstand der ersten und zweiten Oberfläche
- D: Gesamtdicke des Folienelements
- 201: elastisch verformbare, elektrisch nichtleitende erste Schicht
- 202: elastisch verformbare, elektrisch nichtleitende zweite Schicht
- 203: Abstandshalter
- 204: elastisch verformbare, elektrisch leitende erste Leitungen
- 205: elastisch verformbare, elektrisch leitende zweite Leitungen

## Patentansprüche

1. Minimalinvasives Instrument (1) aufweisend einen vergrößerbaren Sensorkopf mit einem elastische Eigenschaften aufweisenden flächigen Sensorelement (7), wobei das Sensorelement (7) ein Folienelement (13) mit einer ersten Schicht (15) und einer mittels Abstandshaltern (19) von der ersten Schicht (15) beabstandeten zweiten Schicht (17) aufweist, wobei das Sensorelement (7) zwischen den Schichten (15,17) des Folienelements (13) angeordnete, dehnungssensitive Widerstandselemente (21) zum Aufnehmen taktiler Stimuli aufweist, **dadurch gekennzeichnet, dass** die Widerstandselemente polymerbasiert sind und der Sensorkopf eine elektromechanische Schnittstelle (47) aufweist, mittels der der Sensorkopf lösbar fest mit dem übrigen Instrument (1) verbindbar ist.

2. Instrument nach dem vorhergehenden Anspruch, **gekennzeichnet durch** eine Spannvorrichtung (41) mittels der das Sensorelement (7) von einem zusammengefalteten Zustand in einen aufgefalteten Zustand und umgekehrt überführbar ist.

3. Instrument nach einem der vorhergehenden Ansprüche, **dadurch gekennzeichnet, dass** ein Hohlraum (23) von dem Sensorelement (7) und einem Schaft (9) des minimalinvasiven Instruments (1) fluiddicht umschlossen ist, wobei ein an eine Fluidstromquelle (33) angeschlossener Fluidpfad in den Hohlraum (23) mündet.

4. Instrument nach einem der vorhergehenden Ansprüche, **dadurch gekennzeichnet, dass** der Sensorkopf einrollbar und ausrollbar ist, wobei ein Einrollen oder ein Ausrollen des Sensorkopfs mittels Rückstellkräften selbsttätig erfolgt.

5. Instrument nach einem der vorhergehenden Ansprüche, **dadurch gekennzeichnet, dass** das Sensorelement (7) Bereiche mit unterschiedlichen taktilen Auflösungen aufweist.

6. Instrument nach einem der vorhergehenden Ansprüche, **dadurch gekennzeichnet, dass** das Instrument (1) ein mit dem Sensorelement (7) verbundenes taktiles Display (45) aufweist, mittels dem während des Vorgangs von dem taktilen Sensor (5) aufgenommene taktile Stimuli anzeigbar sind.

## Claims

1. A minimally invasive instrument (1) comprising an enlargeable sensor head with a flat sensor element (7) having resilient properties, wherein the sensor element (7) has a film element (13) with a first layer (15) and with a second layer (17) distanced from the first layer (15) by means of spacers (19), wherein the sensor element (7) comprises, arranged between the layers (15, 17) of the film element (13), expansionsensitive resistance elements (21) for picking up tactile stimuli, **characterised in that** the resistance elements are polymer-based and the sensor head comprises an electromechanical interface (47), by means of which the sensor head can be securely connected in a detachable manner to the rest of the instrument (1).

2. The instrument according to the preceding claim, **characterised by** a stretching device (41), by means of which the sensor element (7) can be transferred from a folded state into an unfolded state and vice versa.

3. The instrument according to one of the preceding claims, **characterised in that** a cavity (23) is surrounded in a fluid-tight manner by the sensor element (7) and a shaft (9) of the minimally invasive instrument (1), wherein a fluid path connected to a fluid flow source (33) discharges into the cavity (23) .

4. The instrument according to one of the preceding claims, **characterised in that** the sensor head is capable of being rolled up and rolled out, wherein the sensor head is rolled up or rolled out automatically by means of restoring forces.

5. The instrument according to one of the preceding claims, **characterised in that** the sensor element (7) comprises regions having different tactile resolutions.

6. The instrument according to one of the preceding claims, **characterised in that** the instrument (1) comprises a tactile display (45), which is connected to the sensor element (7) and by means of which tactile stimuli picked up by the tactile sensor (5) during the procedure can be displayed.

## Revendications

1. Instrument minimalement invasif (1) présentant une tête de capteur agrandissable avec un élément de capteur (7) plat présentant des propriétés élastiques, dans lequel l'élément de capteur (7) présente un élément de feuille (13) avec une première couche (15) et une seconde couche (17) espacée de la première couche (15) au moyen d'écarteurs (19), dans lequel l'élément de capteur (7) présente des éléments de résistance (21) sensibles à la dilation disposés entre les couches (15, 17) de l'élément de feuille (13) pour l'enregistrement de stimuli tactiles,
**caractérisé en ce que** les éléments de résistance sont à base de polymère et **en ce que** la tête de capteur présente une interface électromécanique (47) grâce à laquelle la tête de capteur peut être reliée de manière détachable et fixe au reste de l'instrument (1) .

2. Instrument selon la revendication précédente, **caractérisé par** un dispositif de serrage (41) grâce auquel il est possible de faire passer l'élément de capteur (7) d'un état plié à un état déplié et vis-versa.

3. Instrument selon l'une des revendications précédentes, **caractérisé en ce qu'**un espace creux (23) est entouré de manière étanche aux fluides par l'élément de capteur (7) et une tige (9) de l'instrument minimalement invasif (1), dans lequel un trajet de fluide raccordé à une source d'écoulement de fluide (33) débouche dans l'espace creux (23).

4. Instrument selon l'une des revendications précédentes, **caractérisé en ce que** la tête de capteur est enroulable et déroulable, dans lequel un enroulement ou un déroulement de la tête de capteur s'effectue automatiquement au moyen de forces de rappel.

5. Instrument selon l'une des revendications précédentes, **caractérisé en ce que** l'élément de capteur (7) présente des zones avec des résolutions tactiles différentes.

6. Instrument selon l'une des revendications précédentes, **caractérisé en ce que** l'instrument (1) présente un afficheur tactile (45) relié à l'élément de capteur (7) grâce auquel il est possible d'afficher des stimuli tactiles enregistrés par le capteur tactile (5) au cours du processus.
